# EUROPEAN PATENT APPLICATION

(11) **EP 4 834 925 A1**
(43) Date of publication of application: **04.11.2026**
(21) Application number: 24911460.4
(22) Date of filing: 27.12.2024
(51) Int. Cl.: C07K 14/705, A61P 35/00, C07K 19/00, A61K 38/00, C07K 14/725

(54) **TRANSGENIC RECOMBINANT IMMUNE CELL SPECIFICALLY TARGETING TUMOR AND USE THEREOF**

(30) Priority: 28.12.2023 CN 202311845904
(71) Applicant: SHANGHAI NK CELLTECH CO., LTD., Shanghai 201318 (CN)
(72) Inventor: WANG, Ting, Shanghai 201318 (CN); ZHOU, Yingli, Shanghai 201318 (CN); ZHANG, Cai, Shanghai 201318 (CN); CHEN, Minhua, Shanghai 201318 (CN)
(74) Representative: Dehns
(86) International application number: PCT/CN2024/143148
(87) International publication number: WO 2025/140540

(57) **Abstract**

Provided is a transgenic recombinant immune cell specifically targeting a tumor. The recombinant immune cell includes a chimeric polypeptide and a chimeric antigen receptor. The chimeric polypeptide includes a first extracellular region having an activity of binding to HLA-G protein, a first transmembrane region, and a first intracellular region. The N-terminus of the first transmembrane region is linked to the C-terminus of the first extracellular region. The N-terminus of the first intracellular region is linked to the C-terminus of the first transmembrane region. The first transmembrane region includes a transmembrane region of *Xenopus tropicalis* Notch receptor protein. The chimeric antigen receptor does not have an HLA-G protein-binding activity. The recombinant immune cell greatly enhances precise recognition of tumor cells by immune cells, reduces the off-target effect of cell therapy, and accurately and effectively distinguishes tumor cells from normal cells, providing an effective target and targeting strategy for the treatment of broad-spectrum tumors (particularly solid tumors).

## Description

### FIELD

The present disclosure relates to the field of biopharmaceuticals, and specifically, to a transgenic recombinant immune cell specifically targeting a tumor, a method for preparing the same, and use thereof. More specifically, the present disclosure relates to a chimeric polypeptide, a first nucleic acid molecule, a first expression vector, a second nucleic acid molecule, a second expression vector, a recombinant immune cell, a pharmaceutical composition, and uses thereof.

### BACKGROUND

With the rapid development of biotechnology, immunotherapy has become one of the primary therapies in the field of cancer treatment. Cancer immunotherapy mainly includes adoptive cell therapy, immunomodulators, tumor vaccines, and immune checkpoint blockade therapy. In the field of adoptive cell therapy, chimeric antigen receptor-modified immune cell therapy, particularly chimeric-antigen receptor T-cell (CAR-T) therapy, is currently highly popular and is a star therapy in this field.

The principle of immune cell therapies represented by CAR-T therapy mainly involves modifying the T cells extracted from a patient with a chimeric antigen receptor by genetic engineering methods to form CAR-T cells. By means of the modified chimeric antigen receptor, these CAR-T cells are capable of specifically recognizing tumor surface-associated antigens (tumor cell markers), thereby targeting and killing tumors. Compared with ordinary immune cells, CAR-T cells exhibit higher targeting capability, killing activity, and persistence. Currently, modified immune cell therapies represented by CAR-T cells targeting CD19 and BCMA have shown significant efficacy in treating hematological tumors such as B-cell lymphomas and are considered one of the most promising approaches for tumor treatment.

However, for lack of targets that can effectively distinguish normal cells from tumor cells, CAR-T cell therapy often inevitably kills some normal cells expressing target protein, which causes damage to normal tissues or leads to continuous activation of CAR-T cells due to a widespread presence of targets, thereby releasing a large number of cytokines and resulting in cytokine storms. Moreover, since the CAR-T cell therapy usually recognizes only a single antigen, tumor cells often escape by losing the antigen recognizable by CAR-T cells through gene mutations, leading to drug resistance. Additionally, the high heterogeneity of tumors makes it difficult for a single target carried by CAR-T cells to comprehensively cover all tumor cells, resulting in incomplete or inadequate tumor treatment. The above key issues have become main obstacles to the development and expansion of the current cell therapy field.

Recent research indicates that attempting to solve the above problems by finding a single molecule that is broad-spectrum yet specific, efficient, and safe as a target for CAR therapy may face significant difficulties, for a reason that a broad-spectrum capability and specificity are like two sides of a coin. A single target can hardly balance both the broad-spectrum capability and the specificity. In current tumor treatment research, no perfect target molecule that possesses the above two major characteristics has been found. Therefore, specific target combinations and tools for recognizing and activating these target combinations are one of the highly promising new directions in current cell therapy for achieving both the broad-spectrum capability and the specificity in the immune cell therapy.

### SUMMARY

The present disclosure aims to solve at least one of the technical problems in the related art at least to some extent.

In a first aspect of the present disclosure, the present disclosure provides a chimeric polypeptide. According to an embodiment of the present disclosure, the chimeric polypeptide includes: a first extracellular region, having an activity of binding to HLA-G protein; a first transmembrane region, including a transmembrane region of *Xenopus tropicalis* Notch receptor protein or an amino acid sequence having at least 80% identity thereto, the N-terminus of the first transmembrane region being linked to the C-terminus of the first extracellular region; and a first intracellular region, the N-terminus of the first intracellular region being linked to the C-terminus of the first transmembrane region. In the chimeric polypeptide of the present disclosure, the transmembrane region of *Xenopus tropicalis* Notch receptor protein is selected as the transmembrane region of the chimeric polypeptide (a synthetic Notch receptor, SynNotch), which can enhance the ability and efficiency of the chimeric polypeptide to activate the transcription of downstream genes.

In a second aspect of the present disclosure, the present disclosure provides a first nucleic acid molecule. According to an embodiment of the present disclosure, the first nucleic acid molecule encodes the chimeric polypeptide described in the first aspect. The first nucleic acid molecule according to an embodiment of the present disclosure can encode and obtain the above chimeric polypeptide.

In a third aspect of the present disclosure, the present disclosure provides a first expression vector. According to an embodiment of the present disclosure, the first expression vector carries the first nucleic acid molecule described in the second aspect. When the above first nucleic acid molecule is linked to an expression vector, the first nucleic acid molecule may be directly or indirectly linked to the control elements on the expression vector, provided that these control elements are capable of controlling the translation, expression, or the like of the first nucleic acid molecule. Naturally, these control elements may originate directly from the vector itself or may be exogenous, i.e., not originating from the vector itself. Of course, the first nucleic acid molecule is operably linked to the control elements.

In a fourth aspect of the present disclosure, the present disclosure provides a second nucleic acid molecule. According to an embodiment of the present disclosure, the second nucleic acid molecule includes a first nucleic acid fragment and a second nucleic acid fragment. The 3' end of the first nucleic acid fragment is linked to the 5' end of the second nucleic acid fragment. The second nucleic acid fragment encodes a chimeric antigen receptor targeting a first molecule. The first nucleic acid fragment binds to a first intracellular region and induces expression of the chimeric antigen receptor. The first intracellular region is the first intracellular region as defined in the chimeric polypeptide in the first aspect. The second nucleic acid molecule of the present disclosure can bind to the first intracellular region in the above chimeric polypeptide, thereby activating and inducing expression of the chimeric antigen receptor.

In a fifth aspect of the present disclosure, the present disclosure provides a second expression vector. According to an embodiment of the present disclosure, the second expression vector carries the second nucleic acid molecule described in the fourth aspect. The second expression vector according to an embodiment of the present disclosure can express the chimeric antigen receptor in the above second nucleic acid molecule.

In a sixth aspect of the present disclosure, the present disclosure provides a recombinant immune cell. According to an embodiment of the present disclosure, the recombinant immune cell carries the first nucleic acid molecule described in the second aspect or the first expression vector described in the third aspect; or expresses the chimeric polypeptide described in the first aspect. Under suitable conditions, the recombinant immune cell according to an embodiment of the present disclosure can express the above chimeric polypeptide on the surface of the recombinant immune cell, which can recognize HLA-G protein. After binding to HLA-G protein, the recombinant immune cell can achieve different types of signal outputs, such as activation of expression of specific genes, particularly activation of expression of a chimeric antigen receptor containing a factor having a therapeutic effect (e.g., for treating tumors), for use in treating diseases such as tumors.

In a seventh aspect of the present disclosure, the present disclosure provides a pharmaceutical composition. According to an embodiment of the present disclosure, the pharmaceutical composition includes the recombinant immune cell described in the sixth aspect. As previously mentioned, cells expressing the above chimeric polypeptide can recognize HLA-G protein. After binding to HLA-G protein, the cells can achieve different types of signal outputs, such as activation and suppression of expression of specific genes. Alternatively, after the contact with HLA-G protein, immune cells expressing the chimeric polypeptide can secrete a factor having a therapeutic effect for use in anti-tumor purposes. Therefore, the pharmaceutical composition including the above recombinant immune cell can be used to target HLA-G protein for prevention and/or treatment of a related disease, such as cancer.

In an eighth aspect of the present disclosure, the present disclosure provides use of the recombinant immune cell described in the sixth aspect or the pharmaceutical composition described in the seventh aspect in the manufacture of a medicament for preventing and/or treating a disease.

### Advantageous Effects:

(1) The recombinant immune cell of the present disclosure greatly enhances precise recognition of tumor cells by immune cells, reduces off-target effects of cell therapy, and accurately and effectively distinguishes tumor cells from normal cells, providing an effective target and targeting strategy for the treatment of broad-spectrum tumors (particularly solid tumors).
(2) The recombinant immune cell of the present disclosure can convert the inhibitory signal transmitted by the binding of HLA-G to ILT2/4 into an activation signal, which not only effectively kills HLA-G-positive tumor cells but also resists suppression from tumor immune microenvironment, reversing immune cell exhaustion.
(3) The engineered multi-targeted recombinant immune cell of the present disclosure can both broadly recognize different types of tumor cells and accurately distinguish them from normal cells, which significantly improves efficiency and precision of killing various types of tumor cells, thus providing a new method for broad-spectrum and specific tumor immunotherapy with broad application prospects.

Additional aspects and advantages of the present disclosure will be provided at least in part in the following description, or will become apparent at least in part from the following description, or can be learned from practicing of the present disclosure.

### BRIEF DESCRIPTION OF THE DRAWINGS

The above and/or additional aspects and advantages of the present disclosure will become more apparent and readily understanded from the following description of embodiments taken in conjunction with the accompanying drawings.
FIG. 1 is a schematic diagram showing the structural composition of a SynNotch receptor in Example 1 of the present disclosure.
FIG. 2 is a schematic diagram showing the structural composition of a CAR receptor in Example 2 of the present disclosure.
FIG. 3 shows results of the killing of double-target-positive K562 cells by Syn-CAR-NK cells in Example 3 of the present disclosure.
FIG. 4 shows results of the killing of single-target-positive Aspc-1 cells by Syn-CAR-NK cells in Example 3 of the present disclosure.
FIG. 5 shows results of the killing of target-negative THLE3 cells by Syn-CAR-NK cells in Example 3 of the present disclosure.
FIG. 6 shows therapeutic results of Syn-CAR-NK cells against subcutaneous xenograft tumors of double-target-positive colon cancer in mice in Example 4 of the present disclosure.
FIG. 7 shows therapeutic results of Syn-CAR-NK cells against subcutaneous xenograft tumors of single-target-positive pancreatic cancer in mice in Example 4 of the present disclosure.

### DETAILED DESCRIPTION

Embodiments of the present disclosure are described in detail below. The embodiments described below are exemplary only, intended solely to explain the present disclosure, and shall not be construed as limiting the present disclosure.

It should be noted that, terms "first" and "second" are only used for descriptive purposes, and cannot be understood as indicating or implying relative importance or implicitly indicating the number of indicated technical features. Therefore, the features associated with "first" and "second" may explicitly or implicitly include at least one of the features. Further, in the description of the present disclosure, "more" or "plurality" means at least two, unless otherwise specified.

### Definitions and General Terminology

To facilitate understanding of the present disclosure, certain technical and scientific terms are specifically defined below. Unless otherwise clearly defined elsewhere in the present disclosure, all other technical and scientific terms used herein have the meanings commonly understood by a person of ordinary skill in the art to which the present disclosure pertains.

As used herein, the terms "comprise" or "include" are open-ended expressions, meaning that they encompass elements specified in the present disclosure but do not exclude additional elements.

As used herein, the terms "optionally", "optional", or "alternative" generally mean that the event or condition subsequently described may but need not occur, and such description includes both scenarios where the event or condition occurs and scenarios where the event or condition does not occur.

As used herein, the terms "identity", "homology", or "similarity", when used to describe an amino acid sequence or a nucleic acid sequence relative to a reference sequence, refer to the percentage of identical amino acids or nucleotides between two amino acid sequences or nucleic acid sequences as determined by conventional methods. See, for example, Ausubel et al., eds. (1995), Current Protocols in Molecular Biology, Chapter 19 (Greene Publishing and Wiley-Interscience, New York); and the ALIGN program (Dayhoff (1978), Atlas of Protein Sequence and Structure 5: Suppl. 3 (National Biomedical Research Foundation, Washington, D.C.)). Many algorithms exist for aligning sequences and determining sequence identity, including: the homology alignment algorithm of Needleman et al. (1970) J. Mol. Biol. 48:443; the local homology algorithm of Smith et al. (1981) Adv. Appl. Math. 2:482; the similarity search method of Pearson et al. (1988) Proc. Natl. Acad. Sci. 85:2444; the Smith-Waterman algorithm (Meth. Mol. Biol. 70:173-187 (1997)); and the BLASTP, BLASTN, and BLASTX algorithms (see Altschul et al. (1990) J. Mol. Biol. 215:403-410). Computer programs utilizing these algorithms are also available and include, but are not limited to: ALIGN or Megalign (DNASTAR) software; WU-BLAST-2 (Altschul et al., Meth. Enzym., 266:460-480 (1996)); GAP, BESTFIT, BLAST (Altschul et al., supra), FASTA, and TFASTA, available in the Genetics Computing Group (GCG) package, version 8, Madison, Wisconsin, USA; and CLUSTAL in the PC/Gene program provided by Intelligenetics, Mountain View, California.

As used herein, the term "at least 80% identity" refers to identity of at least 80% relative to respective reference sequence, which may be 80%, 81%, 82%, 83%, 84%, 85%, 86%, 87%, 88%, 89%, 90%, 91%, 92%, 93%, 94%, 95%, 96%, 97%, 98%, 99%, 99.5%, or 99.9%.

As used herein, the term "expression vector" generally refers to a nucleic acid molecule capable of autonomous replication when inserted into a suitable host, which transfers the inserted nucleic acid molecule into and/or between host cells. The expression vector may include vectors primarily used for inserting DNA or RNA into cells, vectors primarily used for replicating DNA or RNA, and vectors primarily used for expression of transcription and/or translation of DNA or RNA. The expression vector also includes vectors possessing several of the above functions. The expression vector may be a polynucleotide that can be transcribed and translated into a polypeptide when introduced into a suitable host cell. Typically, the expression vector can produce a desired expression product by culturing a suitable host cell containing the expression vector.

As used herein, the term "chimeric antigen receptor (CAR)" is a fusion protein including an extracellular domain capable of binding an antigen, a transmembrane domain derived from a different polypeptide than the extracellular domain, and at least one intracellular domain. The "chimeric antigen receptor (CAR)" is also referred to as "chimeric receptor", "T-body", or "chimeric immune receptor (CIR)." The "extracellular domain capable of binding an antigen" refers to any oligopeptide or polypeptide capable of binding a specific antigen. The "intracellular domain" refers to any oligopeptide or polypeptide known to function as a domain that transmits signals to activate or inhibit intracellular biological processes.

As used herein, the term "recombinant immune cell" generally refers to a cell whose genetic material has been modified, engineered, or recombined using genetic engineering techniques or cell fusion techniques to obtain stably heritable unique characteristics. The term "host cell" refers to a prokaryotic or eukaryotic cell into which a recombinant expression vector can be introduced. The terms "transformed" or "transfected" as used herein refer to introduction of a nucleic acid (e.g., a vector) into a cell by various techniques known in the art. Suitable host cells can be transformed or transfected with the DNA sequences of the present disclosure and can be used for expression and/or secretion of target proteins.

As used herein, the term "pharmaceutical composition" generally refers to a unit dosage form and may be prepared by any of the methods well known in the pharmaceutical field. All methods include the step of combining an active ingredient with a carrier that constitutes one or more accessory ingredients. Typically, a composition is prepared by uniformly and thoroughly combining an active chimeric polypeptide or a recombinant immune cell with a liquid carrier, a finely divided solid carrier, or a combination thereof.

As used herein, the term "pharmaceutically acceptable excipient" may include any solvent, solid excipient, diluent, or other liquid excipient, suitable for a specific desired dosage form. Except to the extent that any conventional excipient is incompatible with the chimeric polypeptide or recombinant immune cell of the present disclosure, e.g., causing any undesirable biological effect or interacting in a harmful manner with any other component of a pharmaceutically acceptable composition, their use is also encompassed within the scope of the present disclosure.

As used herein, the term "administration" refers to introduction of a predetermined amount of a substance into a patient via a suitable route. The recombinant immune cell or pharmaceutical composition of the present disclosure may be administered by any conventional route, as long as they can reach an intended tissue. Various manners of administration are contemplated, including intraperitoneal injection, intravenous injection, intramuscular injection, subcutaneous injection, and the like; however, the present disclosure is not limited to these exemplified manners of administration. Preferably, the composition of the present disclosure is administered via intravenous injection or subcutaneous injection.

As used herein, the term "treatment" or "treating" refers to achieving a desired pharmacological and/or physiological effect. The effect may be prophylactic in terms of fully or partially preventing a disease or symptoms of the disease, and/or therapeutic in terms of partially or fully curing the disease and/or adverse effects caused by the disease. As used herein, the term "treatment" or "treating" covers treatment of a disease in a mammal, particularly a human, and includes: (a) preventing the onset of a disease or condition in an individual who is susceptible but has not yet been diagnosed with the disease; (b) inhibiting the disease, for example, arresting progression of the disease; or (c) alleviating the disease, for example, relieving symptoms associated with the disease. As used herein, the term "treatment" or "treating" encompasses any administration of the recombinant immune cell, the pharmaceutical composition, or the medicament to an individual for treating, curing, alleviating, ameliorating, mitigating, or inhibiting a disease of an individual, including, but not limited to, administering the medicament containing the chimeric polypeptide, the recombinant immune cell, or the pharmaceutical composition described herein to an individual in need thereof.

The term "immune cell" generally refers to a cell capable of mounting an immune response (e.g., an antigen-specific immune response). For example, the immune cell may or already contains an individual cell, a cell line, or a cell culture that includes the isolated nucleic acid and/or the expression vector of the present disclosure or is capable of expressing the chimeric polypeptide and optionally the chimeric antigen receptor described herein. In the present disclosure, the immune cell may include a T cell, a B cell, a natural killer (NK) cell, a macrophage, an NKT cell, a monocyte, a dendritic cell, a granulocyte, a lymphocyte, a leukocyte, and/or a peripheral blood mononuclear cell. As used herein, "carboxyl terminus" and "C-terminus" are synonymous; "amino terminus" and "N-terminus" are synonymous.

### Detailed Description of Transgenic Recombinant Immune Cell Specifically Targeting Tumor, Method for Preparing the Same, and Uses Thereof in the Present Disclosure

The present disclosure provides a chimeric polypeptide, a first nucleic acid molecule, a first expression vector, a second nucleic acid molecule, a second expression vector, a recombinant immune cell, a pharmaceutical composition, and uses thereof, which will be described in detail below.

### Chimeric Polypeptide, First Nucleic Acid Molecule, and First Expression Vector

In a first aspect of the present disclosure, the present disclosure provides a chimeric polypeptide. According to an embodiment of the present disclosure, the chimeric polypeptide includes: a first extracellular region, having an activity of binding to HLA-G protein; a first transmembrane region, including a transmembrane region of *Xenopus tropicalis* Notch receptor protein or an amino acid sequence having at least 80% identity thereto, the N-terminus of the first transmembrane region being linked to the C-terminus of the first extracellular region; and a first intracellular region, the N-terminus of the first intracellular region being linked to the C-terminus of the first transmembrane region. In the chimeric polypeptide of the present disclosure, the transmembrane region of *Xenopus tropicalis* Notch receptor protein is selected as the transmembrane region of the chimeric polypeptide (a synthetic Notch receptor, SynNotch), which can enhance the ability and efficiency of the chimeric polypeptide to activate the transcription of downstream genes.

Human leukocyte antigen-G (HLA-G) is a group of closely linked genes located on the short arm of human chromosome 6. It belongs to a non-classical class I molecule of the major histocompatibility complex (MHC) in humans and is characterized by selective tissue distribution. HLA-G is expressed in some tumor cell lines, tumor biopsy tissues, some infected tissue cells, graft cells after heart transplantation, or the like.

Further, the above chimeric polypeptide may be used to prepare a multi-targeted recombinant immune cell. The recombinant immune cell enables a cell expressing the chimeric polypeptide to recognize HLA-G protein. After binding to HLA-G protein, the recombinant immune cell can achieve different types of signal outputs, such as activation of expression of specific genes, particularly activation of expression of a chimeric antigen receptor containing a factor having a therapeutic effect (e.g., for treating tumors), for use in treating diseases such as tumors.

In particular, the recombinant immune cell for treating tumors can be prepared, which can broadly recognize numerous tumor cells, significantly improving efficiency and precision of killing various types of tumor cells. As a result, the problems of non-specific tumor recognition and incomplete tumor recognition coverage in current clinical applications of cell therapy are overcome, thereby providing a new tumor treatment method with broad application prospects.

According to an embodiment of the present disclosure, the above chimeric polypeptide may further include at least one of the following technical features.

According to an embodiment of the present disclosure, the transmembrane region of *Xenopus tropicalis* Notch receptor protein has the amino acid sequence as set forth in SEQ ID NO: 1.

ILDYGFIGGLGKNITPPDNEEICENEQCAELADNKICNANCNNHACGWDG
GDCSLNFNDPWKNCTQSLQCWKYFNDGKCDSQCNNSGCLYDGFDCQKVEVQCNPL
YDQYCRDHFQDGHCDQGCNNAECEWDGLDCDNMPENLAEGTLLIVVLMPPEKLKN
NSVNFLRELSRVLHTNVVFKKDSKGEYKIYPYYGNEEELKKHHIKKRSAASWSDAPT
AIFSTMKESVLPGRRRRELDQMEVRGSIVYLEIDNRQCYKSSSQCFTSATDVAAFLGA
LATHGNLNIPYKIEAVKSEIVETAKPPPPLYAMFSMLVIPLLIIFVIMVVIVNKKRRR
(SEQ ID NO: 1).

According to an embodiment of the present disclosure, the transmembrane region further includes an epidermal growth factor-like repeat sequence (EGF repeat) and/or an RAM sequence. As a result, basal leak activation of the synthetic receptor SynNotch can be further reduced.

According to an embodiment of the present disclosure, the C-terminus of the epidermal growth factor-like repeat sequence is linked to the N-terminus of the transmembrane region of *Xenopus tropicalis* Notch receptor protein, and/or the C-terminus of the transmembrane region of *Xenopus tropicalis* Notch receptor protein is linked to the N-terminus of the RAM sequence.

According to an embodiment of the present disclosure, the epidermal growth factor-like repeat sequence is the amino acid sequence as set forth in SEQ ID NO: 23.

VVSPCASRPCYNGGTCQFSPEEPFFQCFCPTNFNGLFCH (SEQ ID NO: 23).

According to an embodiment of the present disclosure, the RAM sequence is the amino acid sequence as set forth in SEQ ID NO: 24.

EHGQLWFP (SEQ ID NO: 24).

According to an embodiment of the present disclosure, the first transmembrane region has the amino acid sequence as set forth in SEQ ID NO: 1 or SEQ ID NO: 25, or an amino acid sequence having at least 90% identity thereto.

According to an embodiment of the present disclosure, the first transmembrane region has the amino acid sequence as set forth in SEQ ID NO: 1 or SEQ ID NO: 25.

VVSPCASRPCYNGGTCQFSPEEPFFQCFCPTNFNGLFCHVVSPCASRPCYN
GGTCQFSPEEPFFQCFCPTNFNGLFCHILDYGFIGGLGKNITPPDNEEICENEQCAELAD
NKICNANCNNHACGWDGGDCSLNFNDPWKNCTQSLQCWKYFNDGKCDSQCNNSG

CLYDGFDCQKVEVQCNPLYDQYCRDHFQDGHCDQGCNNAECEWDGLDCDNMPEN
LAEGTLLIVVLMPPEKLKNNSVNFLRELSRVLHTNVVFKKDSKGEYKIYPYYGNEEEL
KKHHIKKRSAASWSDAPTAIFSTMKESVLPGRRRRELDQMEVRGSIVYLEIDNRQCY
KSSSQCFTSATDVAAFLGALATHGNLNIPYKIEAVKSEIVETAKPPPPLYAMFSMLVIPL
LIIFVIMVVIVNKKRRREHGQLWFPEHGQLWFP (SEQ ID NO: 25).

According to an embodiment of the present disclosure, the first extracellular region includes a first binding protein or fragment thereof for binding to HLA-G protein.

In an alternative embodiment of the present disclosure, the first extracellular region may include one or more of the first binding protein or fragment thereof.

According to an embodiment of the present disclosure, the first binding protein or fragment thereof includes at least one of an antibody or functional fragment thereof, or a receptor.

According to an embodiment of the present disclosure, the first binding protein or fragment thereof includes a first binding moiety and/or a second binding moiety. The first binding moiety is an extracellular region of ILT2 protein or active fragment thereof. The second binding moiety is an extracellular region of ILT4 protein or active fragment thereof.

It should be noted that an extracellular region (as referred to as extracellular domain, ECD) of each of ILT2 protein and ILT4 protein can be divided into four domains, named Domain 1 (D1), Domain 2 (D2), Domain 3 (D3), and Domain 4 (D4), respectively.

In an alternative embodiment of the present disclosure, when the first extracellular region includes a plurality of the first binding protein or fragment thereof, each of the plurality of the first binding protein or fragment thereof is independently selected from the first binding moiety or the second binding moiety.

According to an embodiment of the present disclosure, the extracellular region of ILT2 protein is composed of ILT2-D1 domain, ILT2-D2 domain, ILT2-D3 domain, and ILT2-D4 domain. The ILT2-D1 domain has the amino acid sequence as set forth in SEQ ID NO: 2. The ILT2-D2 domain has the amino acid sequence as set forth in SEQ ID NO: 3. The ILT2-D3 domain has the amino acid sequence as set forth in SEQ ID NO: 4. The ILT2-D4 domain has the amino acid sequence as set forth in SEQ ID NO: 5.

PKPTLWAEPGSVITQGSPVTLRCQGGQETQEYRLYREKKTAPWITRIPQELV

KKGQFPIPSITWEHTGRYRCYYGSDTAGRSESSDPLE (SEQ ID NO: 2).

LVVTGAYIKPTLSAQPSPVVNSGGNVTLQCDSQVAFDGFILCKEGEDEHPQ
CLNSQPHARGSSRAIFSVGPVSPSRRWWYRCYAYDSNSPYEWSLPSDLLELLVLG
(SEQ ID NO: 3).

VSKKPSLSVQPGPIVAPEETLTLQCGSDAGYNRFVLYKDGERDFLQLAGAQ
PQAGLSQANFTLGPVSRSYGGQYRCYGAHNLSSEWSAPSD (SEQ ID NO: 4).

PLDILIAGQFYDRVSLSVQPGPTVASGENVTLLCQSQGWMQTFLLTKEGAA
DDPWRLRSTYQSQKYQAEFPMGPVTSAHAGTYRCYGSQSSKPYLLT (SEQ ID NO: 5).

In an exemplary embodiment of the present disclosure, when the first extracellular region includes a plurality of the first binding protein or fragment thereof, the plurality of the first binding protein or fragment thereof may all be the ILT2-D1 domains, or some may be the ILT2-D1 domain and others may be the ILT2-D2 domain.

According to an embodiment of the present disclosure, the extracellular region of ILT2 protein includes, from the N-terminus to the C-terminus, the ILT2-D1 domain, the ILT2-D2 domain, the ILT2-D3 domain, and the ILT2-D4 domain.

According to an embodiment of the present disclosure, the extracellular region of ILT2 protein has the amino acid sequence as set forth in SEQ ID NO: 6.

PKPTLWAEPGSVITQGSPVTLRCQGGQETQEYRLYREKKTAPWITRIPQELV
KKGQFPIPSITWEHTGRYRCYYGSDTAGRSESSDPLELVVTGAYIKPTLSAQPSPVVNS
GGNVTLQCDSQVAFDGFILCKEGEDEHPQCLNSQPHARGSSRAIFSVGPVSPSRRWW
YRCYAYDSNSPYEWSLPSDLLELLVLGVSKKPSLSVQPGPIVAPEETLTLQCGSDAGYN
RFVLYKDGERDFLQLAGAQPQAGLSQANFTLGPVSRSYGGQYRCYGAHNLSSEWSA
PSDPLDILIAGQFYDRVSLSVQPGPTVASGENVTLLCQSQGWMQTFLLTKEGAADDP
WRLRSTYQSQKYQAEFPMGPVTSAHAGTYRCYGSQSSKPYLLT (SEQ ID NO: 6).

According to an embodiment of the present disclosure, the first binding moiety is selected from: at least one of the ILT2-D1 domain, the ILT2-D2 domain, the ILT2-D3 domain, or the ILT2-D4 domain; or the extracellular region of ILT2 protein.

According to an embodiment of the present disclosure, the first binding moiety includes the ILT2-D1 domain and the ILT2-D2 domain. The ILT2-D1 domain is linked to the ILT2-D2 domain.

According to an embodiment of the present disclosure, the C-terminus of the ILT2-D1 domain is linked to the N-terminus of the ILT2-D2 domain, or the N-terminus of the ILT2-D1 domain is linked to the C-terminus of the ILT2-D2 domain.

According to an embodiment of the present disclosure, the first binding moiety is the extracellular region of ILT2 protein.

According to an embodiment of the present disclosure, the extracellular region of ILT4 protein is composed of ILT4-D1 domain, ILT4-D2 domain, ILT4-D3 domain, and ILT4-D4 domain. The ILT4-D1 domain has the amino acid sequence as set forth in SEQ ID NO: 7. The ILT4-D2 domain has the amino acid sequence as set forth in SEQ ID NO: 8. The ILT4-D3 domain has the amino acid sequence as set forth in SEQ ID NO: 9. The ILT4-D4 domain has the amino acid sequence as set forth in SEQ ID NO: 10.

PKPTLWAEPDSVITQGSPVTLSCQGSLEAQEYRLYREKKSASWITRIRPELV
KNGQFHIPSITWEHTGRYGCQYYSRARWSELS (SEQ ID NO: 7).

DPLVLVMTGAYPKPTLSAQPSPVVTSGGRVTLQCESQVAFGGFILCKEGED
EHPQCLNSQPHARGSSRAIFSVGPVSPNRRWSHRCYGYDLNSPYVWSSPSDLLELLVP
GVSKKPSLSV (SEQ ID NO: 8).

QPGPVMAPGESLTLQCVSDVGYDRFVLYKEGERDLRQLPGRQPQAGLSQA
NFTLGPVSRSYGGQYRCYGAHNLSSECSAPSDPLDILIT (SEQ ID NO: 9).

GQIRGTPFISVQPGPTVASGENVTLLCQSWRQFHTFLLTKAGAADAPLRLR
SIHEYPKYQAEFPMSPVTSAHAGTYRCYGSLNSDPYLLSHPSEPLELVVS (SEQ ID NO:
10).

In an exemplary embodiment of the present disclosure, when the first extracellular region includes a plurality of the first binding protein or fragment thereof, the plurality of the first binding protein or fragment thereof may all be the ILT4-D1 domains, or some may be the ILT4-D1 domain and others may be the ILT4-D2 domain.

According to an embodiment of the present disclosure, the extracellular region of ILT4 protein has the amino acid sequence as set forth in SEQ ID NO: 11.

PKPTLWAEPDSVITQGSPVTLSCQGSLEAQEYRLYREKKSASWITRIRPELV
KNGQFHIPSITWEHTGRYGCQYYSRARWSELS(ILT4-D1)DPLVLVMTGAYPKPTLSAQ
PSPVVTSGGRVTLQCESQVAFGGFILCKEGEDEHPQCLNSQPHARGSSRAIFSVGPVSP

NRRWSHRCYGYDLNSPYVWSSPSDLLELLVPGVSKKPSLSV(ILT4-D2)QPGPVMAPG
ESLTLQCVSDVGYDRFVLYKEGERDLRQLPGRQPQAGLSQANFTLGPVSRSYGGQYR
CYGAHNLSSECSAPSDPLDILIT(ILT4-D3)GQIRGTPFISVQPGPTVASGENVTLLCQSW
RQFHTFLLTKAGAADAPLRLRSIHEYPKYQAEFPMSPVTSAHAGTYRCYGSLNSDPY
LLSHPSEPLELVVS (SEQ ID NO: 11).

In an exemplary embodiment of the present disclosure, when the first extracellular region includes a plurality of the first binding protein or fragment thereof, the plurality of the first binding protein or fragment thereof may all be the first binding moieties, may all be the second binding moieties, or some may be the first binding moiety and others the second binding moiety. Specific types are not limited, and all fall within the scope of protection of the present disclosure.

According to an embodiment of the present disclosure, the second binding moiety is selected from: at least one of the ILT4-D1 domain, the ILT4-D2 domain, the ILT4-D3 domain, or the ILT4-D4 domain, or the extracellular region of ILT4 protein.

According to an embodiment of the present disclosure, the second binding moiety includes the ILT4-D1 domain and the ILT4-D2 domain. The ILT4-D1 domain is linked to the ILT4-D2 domain.

According to an embodiment of the present disclosure, the C-terminus of the ILT4-D1 domain is linked to the N-terminus of the ILT4-D2 domain, or the N-terminus of the ILT4-D1 domain is linked to the C-terminus of the ILT4-D2 domain.

According to an embodiment of the present disclosure, the second binding moiety is the extracellular region of ILT4 protein.

According to an embodiment of the present disclosure, when the first extracellular region includes a plurality of the first binding protein or fragment thereof, the first extracellular region further includes a first linker peptide. Any two of the plurality of the first binding protein or fragment thereof are linked with or without the first linker peptide.

As used herein, the expression "any two of the plurality of the first binding protein or fragment thereof are linked with or without the first linker peptide" means that any two of the plurality of the first binding protein or fragment thereof may all be linked via the first linker peptide, any two of the plurality of the first binding protein or fragment thereof may be linked without the first linker peptide, or when three or more of the first binding protein or fragment thereof exist, two of the first binding protein or fragment thereof may be linked via the first linker peptide while another two of the first binding protein or fragment thereof may be linked without the first linker peptide. The specific linking methods are not limited, and all fall within the scope of protection of the present disclosure.

In an alternative embodiment of the present disclosure, when the first extracellular region includes two of the first binding protein or fragment thereof, the first extracellular region includes, from the N-terminus to the C-terminus, one of the first binding protein or fragment thereof, and the other of the first binding protein or fragment thereof.

In an alternative embodiment of the present disclosure, when the first extracellular region includes two of the first binding protein or fragment thereof, the first extracellular region includes, from the N-terminus to the C-terminus, one of the first binding protein or fragment thereof, the first linker peptide, and the other of the first binding protein or fragment thereof.

In an alternative embodiment of the present disclosure, when the first extracellular region includes three of the first binding protein or fragment thereof, the first extracellular region includes, from the N-terminus to the C-terminus, one of the first binding protein or fragment thereof, another one of the first binding protein or fragment thereof, and the other one of the first binding protein or fragment thereof.

In an alternative embodiment of the present disclosure, when the first extracellular region includes three of the first binding protein or fragment thereof, the first extracellular region includes, from the N-terminus to the C-terminus, one of the first binding protein or fragment thereof, another one of the first binding protein or fragment thereof, the first linker peptide, and the other one of the first binding protein or fragment thereof.

In an alternative embodiment of the present disclosure, when the first extracellular region includes three of the first binding protein or fragment thereof, the first extracellular region includes, from the N-terminus to the C-terminus, one of the first binding protein or fragment thereof, the first linker peptide, another one of the first binding protein or fragment thereof, and the other one of the first binding protein or fragment thereof.

In an alternative embodiment of the present disclosure, when the first extracellular region includes three of the first binding protein or fragment thereof, the first extracellular region includes, from the N-terminus to the C-terminus, one of the first binding protein or fragment thereof, the first linker peptide, another one of the first binding protein or fragment thereof, the first linker peptide, and the other one of the first binding protein or fragment thereof.

According to an embodiment of the present disclosure, the first binding protein or fragment thereof includes the first binding moiety and the second binding moiety. The first binding moiety is linked to the second binding moiety.

It should be noted that, the term "linked to" as used herein may refer to direct linkage or indirect linkage. Specific types are not limited, and all fall within the scope of protection of the present disclosure. As an example, "the first binding moiety is linked to the second binding moiety" means that the first binding moiety and the second binding moiety may be directly linked or indirectly linked (e.g., via the first linker peptide).

According to an embodiment of the present disclosure, the first binding protein or fragment thereof includes the first binding moiety and the second binding moiety. The first binding moiety and the second binding moiety are linked via the first linker peptide.

According to an embodiment of the present disclosure, the C-terminus of the first binding moiety is linked to the N-terminus of the first linker peptide and the C-terminus of the first linker peptide is linked to the N-terminus of the second binding moiety, or the C-terminus of the second binding moiety is linked to the N-terminus of the first linker peptide and the C-terminus of the first linker peptide is linked to the N-terminus of the first binding moiety.

According to an embodiment of the present disclosure, the amino acid sequence of the first linker peptide is (GGGGS)n, where n is any integer ranging from 1 to 10.

According to an embodiment of the present disclosure, n is 1, 2, 3 or 4.

According to an embodiment of the present disclosure, the amino acid sequence of the first linker peptide is GGGGS (SEQ ID NO: 22).

According to an embodiment of the present disclosure, the first extracellular region is selected from: ILT2-D1 domain + ILT2-D2 domain + first linker peptide + ILT4-D1 domain + ILT4-D2 domain, ILT4-D1 domain + ILT4-D2 domain + first linker peptide + ILT2-D1 domain + ILT2-D2 domain, the extracellular region of ILT2 protein + first linker peptide + the extracellular region of ILT4 protein, the extracellular region of ILT4 protein + first linker peptide + the extracellular region of ILT2 protein, ILT2-D1 domain + ILT2-D2 domain + first linker peptide + the extracellular region of ILT4 protein, ILT4-D1 domain + ILT4-D2 domain + first linker peptide + the extracellular region of ILT2 protein, ILT2-D1 domain + first linker peptide + ILT4-D1 domain, ILT2-D1 domain + first linker peptide + ILT4-D2 domain, ILT2-D1 domain + first linker peptide + ILT4-D1 domain + ILT4-D2 domain, ILT2-D1 domain + first linker peptide + the extracellular region of ILT4 protein, ILT2-D1 domain + first linker peptide + ILT2-D1 domain, ILT2-D1 domain + first linker peptide + ILT2-D2 domain, ILT2-D1 domain + first linker peptide + ILT2-D1 domain + ILT2-D2 domain, ILT2-D1 domain + first linker peptide + the extracellular region of ILT2 protein, ILT4-D1 domain + first linker peptide + ILT4-D1 domain, ILT4-D1 domain + first linker peptide + ILT4-D2 domain, ILT4-D1 domain + first linker peptide + ILT4-D1 domain + ILT4-D2 domain, ILT4-D1 domain + first linker peptide + the extracellular region of ILT4 protein, ILT4-D1 domain + first linker peptide + ILT2-D1 domain, ILT4-D1 domain + first linker peptide + ILT2-D2 domain, ILT4-D1 domain + first linker peptide + ILT2-D1 domain + ILT2-D2 domain, ILT4-D1 domain + first linker peptide + the extracellular region of ILT2 protein, ILT2-D2 domain + first linker peptide + ILT4-D1 domain, ILT2-D2 domain + first linker peptide + ILT4-D2 domain, ILT2-D2 domain + first linker peptide + ILT4-D1 domain + ILT4-D2 domain, ILT2-D2 domain + first linker peptide + the extracellular region of ILT4 protein, ILT2-D2 domain + first linker peptide + ILT2-D1 domain, ILT2-D2 domain + first linker peptide + ILT2-D2 domain, ILT2-D2 domain + first linker peptide + ILT2-D1 domain + ILT2-D2 domain, ILT2-D2 domain + first linker peptide + the extracellular region of ILT2 protein, ILT4-D2 domain + first linker peptide + ILT4-D1 domain, ILT4-D2 domain + first linker peptide + ILT4-D2 domain, ILT4-D2 domain + first linker peptide + ILT4-D1 domain + ILT4-D2 domain, ILT4-D2 domain + first linker peptide + the extracellular region of ILT4 protein, ILT4-D2 domain + first linker peptide + ILT2-D1 domain, ILT4-D2 domain + first linker peptide + ILT2-D2 domain, ILT4-D2 domain + first linker peptide + ILT2-D1 domain + ILT2-D2 domain, ILT4-D2 domain + first linker peptide + the extracellular region of ILT2 protein, ILT2-D1 domain + ILT2-D2 domain + first linker peptide + ILT4-D1 domain, ILT2-D1 domain + ILT2-D2 domain + first linker peptide + ILT4-D2 domain, ILT2-D1 domain + ILT2-D2 domain + first linker peptide + ILT4-D1 domain + ILT4-D2 domain, ILT2-D1 domain + ILT2-D2 domain + first linker peptide + the extracellular region of ILT4 protein, ILT2-D1 domain + ILT2-D2 domain + first linker peptide + ILT2-D1 domain, ILT2-D1 domain + ILT2-D2 domain + first linker peptide + ILT2-D2 domain, ILT2-D1 domain + ILT2-D2 domain + first linker peptide + ILT2-D1 domain + ILT2-D2 domain, ILT2-D1 domain + ILT2-D2 domain + first linker peptide + the extracellular region of ILT2 protein, ILT4-D1 domain + ILT4-D2 domain + first linker peptide + ILT4-D1 domain, ILT4-D1 domain + ILT4-D2 domain + first linker peptide + ILT4-D2 domain, ILT4-D1 domain + ILT4-D2 domain + first linker peptide + ILT4-D1 domain + ILT4-D2 domain, ILT4-D1 domain + ILT4-D2 domain + first linker peptide + the extracellular region of ILT4 protein, ILT4-D1 domain + ILT4-D2 domain + first linker peptide + ILT2-D1 domain, ILT4-D1 domain + ILT4-D2 domain + first linker peptide + ILT2-D2 domain, ILT4-D1 domain + ILT4-D2 domain + first linker peptide + ILT2-D1 domain + ILT2-D2 domain, ILT4-D1 domain + ILT4-D2 domain + first linker peptide + the extracellular region of ILT2 protein, the extracellular region of ILT2 protein + first linker peptide + ILT4-D1 domain, the extracellular region of ILT2 protein + first linker peptide + ILT4-D2 domain, the extracellular region of ILT2 protein + first linker peptide + ILT4-D1 domain + ILT4-D2 domain, the extracellular region of ILT2 protein + first linker peptide + the extracellular region of ILT4 protein, the extracellular region of ILT2 protein + first linker peptide + ILT2-D1 domain, the extracellular region of ILT2 protein + first linker peptide + ILT2-D2 domain, the extracellular region of ILT2 protein + first linker peptide + ILT2-D1 domain + ILT2-D2 domain, the extracellular region of ILT2 protein + first linker peptide + the extracellular region of ILT2 protein, the extracellular region of ILT4 protein + first linker peptide + ILT4-D1 domain, the extracellular region of ILT4 protein + first linker peptide + ILT4-D2 domain, the extracellular region of ILT4 protein + first linker peptide + ILT4-D1 domain + ILT4-D2 domain, the extracellular region of ILT4 protein + first linker peptide + the extracellular region of ILT4 protein, the extracellular region of ILT4 protein + first linker peptide + ILT2-D1 domain, the extracellular region of ILT4 protein + first linker peptide + ILT2-D2 domain, the extracellular region of ILT4 protein + first linker peptide + ILT2-D1 domain + ILT2-D2 domain, or the extracellular region of ILT4 protein + first linker peptide + the extracellular region of ILT2 protein.

It should be noted that the linking order for the above first extracellular region is described from the N-terminus to the C-terminus. As an example, "the first extracellular region is ILT2-D1 domain + ILT2-D2 domain + first linker peptide + ILT4-D1 domain + ILT4-D2 domain" means that the first extracellular region includes, from the N-terminus to the C-terminus, the ILT2-D1 domain, the ILT2-D2 domain, the first linker peptide, the ILT4-D1 domain, and the ILT4-D2 domain.

According to an embodiment of the present disclosure, the first extracellular region is selected from: 1) the extracellular region of ILT2 protein; 2) the extracellular region of ILT4 protein; 3) ILT2-D1 domain and ILT2-D2 domain, from the N-terminus to the C-terminus; 4) ILT4-D1 domain and ILT4-D2 domain, from the N-terminus to the C-terminus; 5) ILT2-D1 domain, ILT2-D2 domain, the first linker peptide, ILT4-D1 domain, and ILT4-D2 domain, from the N-terminus to the C-terminus; or 6) ILT4-D1 domain, ILT4-D2 domain, the first linker peptide, ILT2-D1 domain, and ILT2-D2 domain, from the N-terminus to the C-terminus.

According to an embodiment of the present disclosure, the first intracellular region includes at least one of a transcriptional activator, a transcriptional repressor, a transcription factor, a site-specific nuclease, a recombinase, an activating immune receptor intracellular domain, or an inhibitory immune receptor intracellular domain.

According to an embodiment of the present disclosure, the first intracellular region includes at least one of GaL4-VP64, GaL4-VP16, tetR-VP64, ZFHD1-VP64, Gal4-KRAB, HAP1-VP16, or LexA-VP64.

According to an embodiment of the present disclosure, Gal4-VP64 has the amino acid sequence as set forth in SEQ ID NO: 12.

MKLLSSIEQACDICRLKKLKCSKEKPKCAKCLKNNWECRYSPKTKRSPLTR
AHLTEVESRLERLEQLFLLIFPREDLDMILKMDSLQDIKALLTGLFVQDNVNKDAVTD
RLASVETDMPLTLRQHRISATSSSEESSNKGQRQLTVSAAAGGSGGSGGSDALDDFDL
DMLGSDALDDFDLDMLGSDALDDFDLDMLGSDALDDFDLDMLGS (SEQ ID NO: 12).

In a second aspect of the present disclosure, the present disclosure provides a first nucleic acid molecule. According to an embodiment of the present disclosure, the first nucleic acid molecule encodes the chimeric polypeptide described in the first aspect. The first nucleic acid molecule according to an embodiment of the present disclosure can encode and obtain the above chimeric polypeptide.

According to an embodiment of the present disclosure, the first nucleic acid molecule is DNA.

It should be noted that, for the first nucleic acid molecule mentioned herein, those skilled in the art should understand that it actually includes either one or both strands of a complementary double strand. For convenience, in the present disclosure, although only one strand is provided in most cases, the complementary strand is also disclosed. Further, molecular sequences in the present disclosure include both DNA and RNA forms, and disclosure of one form implies disclosure of the other form.

In a third aspect of the present disclosure, the present disclosure provides a first expression vector. According to an embodiment of the present disclosure, the first expression vector carries the first nucleic acid molecule described in the second aspect. When the above first nucleic acid molecule is linked to an expression vector, the first nucleic acid molecule may be directly or indirectly linked to the control elements on the expression vector, provided that these control elements are capable of controlling translation, expression, or the like of the first nucleic acid molecule. Naturally, these control elements may originate directly from the vector itself or may be exogenous, i.e., not originating from the vector itself. Of course, the first nucleic acid molecule is operably linked to the control elements.

As used herein, "operably linked" refers to linking an exogenous gene to an expression vector, such that the control elements within the expression vector, e.g., a transcriptional control sequence and a translational control sequence, can perform their intended functions of regulating transcription and translation of the exogenous gene. Commonly used expression vectors may include plasmids, bacteriophages, and the like. According to some specific embodiments of the present disclosure, after the vector is introduced into an appropriate recipient cell (also referred to as a receptor cell or a host cell), expression of the above chimeric polypeptide can be effectively achieved under mediation of a regulatory system.

According to an embodiment of the present disclosure, the first expression vector is a eukaryotic expression vector, a prokaryotic expression vector, a virus, or a bacteriophage.

According to an embodiment of the present disclosure, the first expression vector is a plasmid expression vector.

### Second Nucleic Acid Molecule and Second Expression Vector

In a fourth aspect of the present disclosure, the present disclosure provides a second nucleic acid molecule. According to an embodiment of the present disclosure, the second nucleic acid molecule includes a first nucleic acid fragment and a second nucleic acid fragment. The 3' end of the first nucleic acid fragment is linked to the 5' end of the second nucleic acid fragment. The second nucleic acid fragment encodes a chimeric antigen receptor targeting a first molecule. The first nucleic acid fragment binds to a first intracellular region and induces expression of the chimeric antigen receptor. The first intracellular region is the first intracellular region as defined in the chimeric polypeptide in the first aspect. The second nucleic acid molecule of the present disclosure can bind to the first intracellular region in the above chimeric polypeptide, activating and inducing expression of the chimeric antigen receptor.

Further, the above second nucleic acid molecule and the above first nucleic acid molecule can be used to prepare a multi-targeted recombinant immune cell. The recombinant immune cell enables a cell expressing the chimeric polypeptide to recognize HLA-G protein. After binding to HLA-G protein, the recombinant immune cell can achieve different types of signal outputs, such as activation of expression of specific genes, particularly activation of expression of a chimeric antigen receptor containing a factor having a therapeutic effect (e.g., for treating tumors), for use in treating diseases such as tumors. In particular, the recombinant immune cell for treating tumors can be prepared, which can broadly recognize numerous tumor cells, significantly improving the efficiency and precision of killing various types of tumor cells. As a result, the problems of non-specific tumor recognition and incomplete tumor recognition coverage in current clinical applications of cell therapy are overcome, thereby providing a new tumor treatment method with broad application prospects.

It should be noted that the term "inducing expression of the chimeric antigen receptor" means enabling the second nucleic acid fragment to encode and obtain the chimeric antigen receptor.

According to an embodiment of the present disclosure, the chimeric antigen receptor includes: a second extracellular region, having a binding activity to the first molecule, the first molecule being not HLA-G protein; a second transmembrane region, the N-terminus of the second transmembrane region being linked to the C-terminus of the second extracellular region; and a second intracellular region, the N-terminus of the second intracellular region being linked to the C-terminus of the second transmembrane region.

According to an embodiment of the present disclosure, the first molecule includes at least one of a tumor antigen, a virus, a bacterium, an endotoxin, an antibody, a cell receptor, or a ligand of a cell receptor.

As used herein, the term "tumor antigen" generally refers to antigenic substances that newly appear or are overexpressed during the occurrence and development process of tumors. According to classification based on tumor antigen specificity, tumor antigens are divided into tumor-specific antigens (TSAs) and tumor-associated antigens (TAAs). TSA is unique to tumor cells or exists only in certain tumor cells but not in normal cells. TAA is not unique to tumor cells and also exists in normal cells and other tissues, but its level is significantly increased during cell carcinogenesis. Examples include, but are not limited to, PD-L1, PD-1, TGF-β, CEA, GD2, and GD3.

As used herein, the term "cell receptor" or "receptor" should be interpreted broadly, and may refer to a molecule that is located on the cell membrane and can recognize and bind to various extracellular signaling molecules (ligands), including but not limited to, a growth factor receptor (e.g., VEGF receptor), NKG2D polypeptide (receptor for MICA, MICB, and ULBP1-6), a cytokine receptor (e.g., IL-13 receptor and IL-2 receptor), an epidermal growth factor (EGF) receptor, Her2, CD27, a natural cytotoxicity receptor (NCR) (e.g., NKp30 (NCR3/CD337) polypeptide (receptor for HLA-B-associated transcript 3 (BAT3) and B7-H6), a T-cell antigen receptor, a dihydrofolate receptor, a chimeric cytokine receptor, an Fc receptor, an extracellular matrix receptor (e.g., integrin), a cell adhesion receptor (e.g., cadherin), an immunoregulatory receptor (including a positive co-receptor (e.g., CD28) and a negative (immunosuppressive) co-receptor (e.g., PD1)), and a receptor for immunoregulatory molecules (e.g., TGFβ).

As used herein, the term "ligand of a cell receptor" should be interpreted broadly and may refer to a chemical substance that can bind to and interact with a cell membrane receptor, and elicit a specific biological effect, such as a polypeptide, a nucleic acid, glycoprotein, a small molecule, carbohydrate, lipid, glycolipid, lipoprotein, and lipopolysaccharide, including but not limited to a cytokine (e.g., IL-13), a growth factor (e.g., heregulin and a vascular endothelial growth factor (VEGF)), a peptide hormone, an integrin-binding peptide (e.g., a peptide containing a sequence Arg-Gly-Asp), and N-glycans.

Exemplarily, the ligand is VEGF and the receptor is a VEGF receptor; or the ligand is heregulin and the receptor is HER2.

As used herein, the term "cytokine" should be interpreted broadly and may refer to a class of proteins or small polypeptide molecules capable of transmitting information between cells and possessing immunomodulatory and effector functions, such as IL-10. The term "cytokine receptor" should be interpreted broadly and may refer to a receptor on the cell surface that is capable of binding to a cytokine, such as IL-10R.

According to an embodiment of the present disclosure, the tumor antigen includes at least one of a tumor-associated antigen or a tumor-specific antigen.

According to an embodiment of the present disclosure, the tumor antigen is a tumor-specific antigen.

According to an embodiment of the present disclosure, the first molecule includes at least one of MICA, MICB, ULBPs, B7H6, B7H3, GFP, eGFP, CD19, ALPPL2, BCMA, SIRPα, CD1a, CD1b, CD1c, CD1d, CD1e, CD2, CD3d, CD3e, CD3g, CD4, CD5, CD7, CD8a, CD8b, CD20, CD21, CD22, CD23, CD25, CD27, CD28, CD30, CD33, CD34, CD38, CD40, CD44, CD44v6, CD45, CD48, CD51, CD52, CD56, CD59, CD66, CD70, CD71, CD72, CD73, CD74, CD79A, CD79B, CD80, CD86, CD94, CD95, CD123, CD133, CD134, CD140, CD152, CD154, CD158, CD178, CD181, CD182, CD183, CD200, CD210, CD221, CD246, CD252, CD253, CD261, CD262, CD269, CD273, CD274, CD276, CD279, CD295, CD339, CD340, EGFR, EGFR VIII, HER2, FGFR2, AFP, CA125, MSLN, GPC3, CEA, CLDN1, CLDN3, CLDN6, CLDN18.1, CLDN18.2, EpCAM, PSCA, GD2, GD3, IL-13, IL-13RA2, ROR1, MUC-1, PSMA, MAGEA1, 4-1BB, 5T4, BAFF, CA242, CA-IX, MET, CCR4, CNTO888, FAP, MORAb-009, EPHA2, VEGF-A, VEGFR-1, or VEGFR-2.

According to an embodiment of the present disclosure, the second extracellular region includes a second binding protein or fragment thereof for binding to the first molecule.

According to an embodiment of the present disclosure, the second binding protein or fragment thereof includes at least one of an antibody or functional fragment thereof, a receptor, a receptor ligand, or a cell adhesion molecule.

According to an embodiment of the present disclosure, the second binding protein or fragment thereof is an extracellular region of an activating receptor expressed on the surface of an immune cell, or an antibody or a fragment thereof binding to the first molecule.

According to an embodiment of the present disclosure, the antibody or fragment thereof binding to the first molecule is a single-chain antibody.

According to an embodiment of the present disclosure, the activating receptor is a receptor expressed on the surface of NK cells.

According to an embodiment of the present disclosure, the activating receptor is selected from at least one of NKG2D, NKp30, NKp44, NKp46, DNAM-1, PD-1, TIGIT, NKG2A, NKG2B, NKG2C, NKG2E, NKG2H, CD16, NKp80, CD226, CD160, CD161, CD96, PVRIG, SLAM, CD200R, CD49a, TIM-3, LAG-3, CD112R, KIR2DS1, KIR2DS2, KIR2DS4, KIR2DL1, KIR2DL2, KIR2DL3, KIR3DL1, KIR3DL2, LIR1, LIR2, SIGLE3, SIGLE7, SIGLE9, or KLRG1.

According to an embodiment of the present disclosure, the activating receptor is selected from NKG2D and/or NKp30.

According to an embodiment of the present disclosure, the second binding protein or fragment thereof includes a third binding moiety and/or a fourth binding moiety. The third binding moiety is an extracellular region of NKG2D or active fragment thereof. The fourth binding moiety is an extracellular region of NKp30 or active fragment thereof.

According to an embodiment of the present disclosure, the extracellular region of NKG2D has the amino acid sequence as set forth in SEQ ID NO: 13.

SLFNQEVQIPLTESYCGPCPKNWICYKNNCYQFFDESKNWYESQASCMSQ
NASLLKVYSKEDQDLLKLVKSYHWMGLVHIPTNGSWQWEDGSILSPNLLTIIEMQKG
DCALYASSFKGYIENCSTPNTYICMQRTV (SEQ ID NO: 13).

According to an embodiment of the present disclosure, the extracellular region of NKp30 has the amino acid sequence as set forth in SEQ ID NO: 14.

LWVSQPPEIRTLEGSSAFLPCSFNASQGRLAIGSVTWFRDEVVPGKEVRNG
TPEFRGRLAPLASSRFLHDHQAELHIRDVRGHDASIYVCRVEVLGLGVGTGNGTRLV
VEKEHPQLGAGTV (SEQ ID NO: 14).

According to an embodiment of the present disclosure, the third binding moiety has the amino acid sequence as set forth in SEQ ID NO: 13 or an amino acid sequence having at least 90% identity thereto.

According to an embodiment of the present disclosure, the fourth binding moiety has the amino acid sequence as set forth in SEQ ID NO: 14 or an amino acid sequence having at least 90% identity thereto.

According to an embodiment of the present disclosure, the second extracellular region further includes a second linker peptide. The third binding moiety and the fourth binding moiety are linked via the second linker peptide.

According to an embodiment of the present disclosure, the C-terminus of the third binding moiety is linked to the N-terminus of the second linker peptide and the C-terminus of the second linker peptide is linked to the N-terminus of the fourth binding moiety, or the C-terminus of the fourth binding moiety is linked to the N-terminus of the second linker peptide and the C-terminus of the second linker peptide is linked to the N-terminus of the third binding moiety.

According to an embodiment of the present disclosure, the amino acid sequence of the second linker peptide is (GGGGS)n, where n is any integer ranging from 0 to 10.

According to an embodiment of the present disclosure, n is 0, 1, 2, 3, or 4.

According to an embodiment of the present disclosure, the amino acid sequence of the second linker peptide is GGGGS.

According to an embodiment of the present disclosure, the second extracellular region further includes a hinge region.

According to an embodiment of the present disclosure, the C-terminus of the third binding moiety is linked to the N-terminus of the second linker peptide, the C-terminus of the second linker peptide is linked to the N-terminus of the fourth binding moiety, and the C-terminus of the fourth binding moiety is linked to the N-terminus of the hinge region, or the C-terminus of the fourth binding moiety is linked to the N-terminus of the second linker peptide, the C-terminus of the second linker peptide is linked to the N-terminus of the third binding moiety, and the C-terminus of the third binding moiety is linked to the N-terminus of the hinge region.

According to an embodiment of the present disclosure, the hinge region includes at least one of a hinge region of CD8α molecule or variant thereof, or a hinge region of an immunoglobulin or variant thereof.

As used herein, the term "immunoglobulin" refers to a globulin that possesses antibody (Ab) activity or a chemical structure similar to that of an antibody molecule, which is a tetrapeptide chain structure formed by two identical light chains and two identical heavy chains that are linked by interchain disulfide bonds. The "immunoglobulin" includes immunoglobulin G (IgG), immunoglobulin A (IgA), immunoglobulin M (IgM), immunoglobulin D (IgD), and immunoglobulin E (IgE).

According to an embodiment of the present disclosure, the hinge region has the amino acid sequence as set forth in SEQ ID NO: 15.

GGGGSGGGGDESKYGPPCPPCPAPEFLGGPSVFLFPPKPKDTLMISRTPEVT
CVVVDVSQEDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVSLTCLVKGFY
PSDIAVEWESNGQPENNYKTTPPVLDSDGSFFLYSRLTVDKSRWQEGNVFSCSVMHE
ALHNHYTQKSLSLSLGK (SEQ ID NO: 15).

According to an embodiment of the present disclosure, the second transmembrane region is selected from at least one of a transmembrane region of CD8α molecule, a transmembrane region of CD28 molecule, a transmembrane region of CD3ζ molecule, a transmembrane region a CD4 molecule, a transmembrane region of CD16 molecule, a transmembrane region of 4-1BB molecule, a transmembrane region of OX40 molecule, a transmembrane region of ICOS molecule, a transmembrane region of CTLA-4 molecule, a transmembrane region of PD-1 molecule, a transmembrane region of LAG-3 molecule, a transmembrane region of 2B4 molecule, a transmembrane region of NKG2D molecule, a transmembrane region of DNAM-1 molecule, a transmembrane region of NKp44 molecule, a transmembrane region of NKp46 molecule, a transmembrane region of KIR2DS1 molecule, a transmembrane region of KIR2DS2 molecule, a transmembrane region of KIR2DS4 molecule, or a transmembrane region of BTLA molecule.

According to an embodiment of the present disclosure, the second transmembrane region is a transmembrane region of CD8α molecule.

According to an embodiment of the present disclosure, the second transmembrane region has the amino acid sequence as set forth in SEQ ID NO: 16.

IYIWAPLAGTCGVLLLSLVITLYC (SEQ ID NO: 16).

According to an embodiment of the present disclosure, the second intracellular region includes an intracellular signaling domain and a co-stimulatory domain.

According to an embodiment of the present disclosure, the intracellular signaling domain is selected from at least one of an intracellular signaling domain of CD3ζ molecule or an intracellular signaling domain of FcεRIγ molecule.

According to an embodiment of the present disclosure, the co-stimulatory domain is selected from at least one of an intracellular signaling domain of 4-1BB molecule, an intracellular signaling domain of CD28 molecule, an intracellular signaling domain of CD27 molecule, an intracellular signaling domain of CD40 molecule, an intracellular signaling domain of OX40 molecule, an intracellular signaling domain of ICOS molecule, an intracellular signaling domain of DAP10 molecule, an intracellular signaling domain of DAP12 molecule, or an intracellular signaling domain of DNAM-1 molecule.

According to an embodiment of the present disclosure, the second intracellular region has the amino acid sequence as set forth in SEQ ID NO: 17.

KRGRKKLLYIFKQPFMRPVQTTQEEDGCSCRFPEEEEGGCELRVKFSRSAD
APAYQQGQNQLYNELNLGRREEYDVLDKRRGRDPEMGGKPRRKNPQEGLYNELQK
DKMAEAYSEIGMKGERRRGKGHDGLYQGLSTATKDTYDALHMQALPPR (SEQ ID
NO: 17).

According to an embodiment of the present disclosure, the second nucleic acid fragment has the nucleotide sequence as set forth in SEQ ID NO: 18.

CTGTGGGTGAGCCAACCCCCCGAGATCAGAACCCTGGAGGGCAGCAGC
GCCTTCCTGCCCTGCAGCTTCAACGCTAGCCAAGGCCGGCTGGCCATCGGCAGCG
TGACCTGGTTCAGAGACGAGGTGGTGCCCGGCAAAGAGGTGAGAAACGGCACCC
CCGAGTTCAGAGGCAGACTGGCCCCCCTGGCTAGCAGCAGATTCCTGCACGACCA

CCAAGCCGAGCTGCACATCAGAGACGTGAGAGGCCACGACGCTAGCATCTACGTG
TGCAGAGTGGAGGTGCTGGGCCTGGGCGTGGGCACCGGCAACGGCACAAGACTG
GTGGTGGAGAAGGAACACCCTCAGCTGGGCGCTGGCACAGTGGGGGGGCGGC
AGCGGCGGGGGCGGCAGCGGCGGGGGCGGAGCAGCCTGTTCAACCAAGAGGT
GCAGATCCCCCTGACCGAGAGCTACTGCGGCCCCTGCCCCAAGAACTGGATCTGC
TACAAGAACAACTGCTATCAGTTCTTCGACGAGAGCAAAAACTGGTACGAGAGCC
AAGCTAGCTGCATGAGCCAAAACGCTAGCCTGCTGAAGGTGTACAGCAAGGAGG
ACCAAGACCTGCTGAAGCTGGTGAAGAGCTACCACTGGATGGGCCTGGTGCACAT
CCCCACCAACGGCAGCTGGCAGTGGGAGGACGGCAGCATCCTGAGCCCCAACCT
GCTGACCATCATCGAGATGCAGAAGGGCGACTGCGCCCTGTACGCTAGCAGCTTC
AAGGGCTACATCGAGAACTGCAGCACCCCCAACACCTACATCTGTATGCAAAGAA
CCGTCGGCGGGGGCGGGAGCGGCGGGGGGGCGACGAAAGCAAGTACGGGCCT
CCTTGTCCCCCCTGTCCCGCCCCCGAGTTCCTGGGCGGCCCTAGCGTGTTCCTGTT
CCCCCCCAAGCCCAAGGACACCCTGATGATCAGCAGAACCCCCGAGGTGACCTGC
GTGGTCGTGGACGTGAGCCAAGAGGACCCCGAGGTGCAGTTCAACTGGTACGTG
GACGGCGTGGAGGTGCACAACGCCAAGACCAAGCCTAGAGAGGAGCAGTTCAAC
AGCACCTACAGAGTGGTGAGCGTGCTGACCGTGCTGCACCAAGACTGGCTGAAC
GGCAAGGAGTACAAGTGCAAGGTGAGCAACAAGGGCCTGCCTAGCAGCATCGAG
AAGACCATCAGCAAGGCCAAGGGGCAGCCTAGAGAGCCCCAAGTGTACACCCTG
CCCCCTAGCCAAGAGGAGATGACCAAGAACCAAGTGAGCCTGACCTGCCTGGTG
AAGGGCTTCTACCCTAGCGACATCGCCGTGGAGTGGGAGAGCAACGGGCAGCCCG
AGAACAACTACAAGACCACCCCCCCCGTGCTGGACAGCGACGGCAGCTTCTTCCT
GTACAGCAGACTGACCGTGGACAAGAGCAGATGGCAAGAGGGCAACGTGTTCAG
CTGCAGCGTGATGCACGAGGCCCTGCACAACCACTACACACAGAAGAGCCTGAG
CCTGAGCCTGGGCAAGATCTACATCTGGGCTCCCCTGGCCGGCACCTGCGGCGTG
CTGCTCCTGAGCCTGGTGATCACCCTGTACTGCAAGAGAGGCAGAAAGAAGCTGC
TGTACATCTTCAAGCAGCCCTTCATGAGACCCGTGCAGACCACCCAAGAGGAGGA
CGGCTGCAGCTGCAGATTCCCCGAGGAAGAGGAGGGCGGCTGCGAGCTGAGAGT
GAAGTTCAGCAGAAGCGCCGACGCCCCCGCCTATCAGCAAGGGCAGAATCAGCTG
TACAACGAGCTGAACCTGGGCAGAAGAGAGGAGTACGACGTGCTGGACAAGAGA

AGAGGCAGAGACCCCGAGATGGGCGGCAAGCCTAGAAGAAAGAACCCCCAAGA
GGGCCTGTATAATGAGCTGCAGAAGGACAAGATGGCCGAGGCCTACAGCGAGATC
GGCATGAAGGGCGAGAGACGGAGAGGCAAGGGCCACGACGGCCTGTACCAAGGC
CTGAGCACCGCCACCAAGGACACCTACGACGCCCTGCACATGCAAGCCCTGCCCC
CTAGA (SEQ ID NO: 18).

According to an embodiment of the present disclosure, the second nucleic acid molecule further includes a third nucleic acid fragment encoding a signal peptide. The 3' end of the first nucleic acid fragment is linked to the 5' end of the third nucleic acid fragment. The 3' end of the third nucleic acid fragment is linked to the 5' end of the second nucleic acid fragment.

According to an embodiment of the present disclosure, the signal peptide is selected from at least one of a signal peptide of CD8α molecule, a signal peptide of an IgG molecule, or a signal peptide of CD28 molecule.

According to an embodiment of the present disclosure, the signal peptide has the amino acid sequence as set forth in SEQ ID NO: 19.

MALPVTALLLPLALLLHAARP (SEQ ID NO: 19).

According to an embodiment of the present disclosure, the third nucleic acid fragment has the nucleotide sequence as set forth in SEQ ID NO: 20.

ATGGCTCTGCCCGTGACAGCTCTGCTCCTGCCTCTCGCCCTCCTCCTGCA
CGCCGCTAGACCC (SEQ ID NO:20).

In an alternative embodiment of the present disclosure, the intracellular domain is GaL4-VP64, and the first nucleic acid fragment is a UAS-minimal-CMV sequence. The UAS-minimal-CMV sequence has the nucleic acid sequence as set forth in SEQ ID NO: 21.

According to an embodiment of the present disclosure, the first nucleic acid fragment has the nucleotide sequence as set forth in SEQ ID NO: 21.

GGAGCACTGTCCTCCGAACGTCGGAGCACTGTCCTCCGAACGTCGGAG
CACTGTCCTCCGAACGTCGGAGCACTGTCCTCCGAACGGAGCATGTCCTCCGAAC
GTCGGAGCACTGTCCTCCGAACGACTAGTTAGGCGTGTACGGTGGGAGGCCTATAT
AAGCAGAGCTCGTTTAGTGAACCGTCAGATCGCCTGGAGACGCCATCCACGCTGT
TTTGACCTCCATAGAAGACACCGGGACCGATCCAGC (SEQ ID NO: 21).

In a fifth aspect of the present disclosure, the present disclosure provides a second expression vector. According to an embodiment of the present disclosure, the second expression vector carries the second nucleic acid molecule described in the fourth aspect. The second expression vector according to an embodiment of the present disclosure can express the chimeric antigen receptor in the above second nucleic acid molecule.

According to an embodiment of the present disclosure, the second expression vector is a eukaryotic expression vector, a prokaryotic expression vector, a virus, or a bacteriophage.

According to an embodiment of the present disclosure, the second expression vector is a plasmid expression vector.

### Recombinant Immune Cell

In a sixth aspect of the present disclosure, the present disclosure provides a recombinant immune cell. According to an embodiment of the present disclosure, the recombinant immune cell carries the first nucleic acid molecule described in the second aspect or the first expression vector described in the third aspect; or expresses the chimeric polypeptide described in the first aspect. Under suitable conditions, the recombinant immune cell according to an embodiment of the present disclosure can express the above chimeric polypeptide on the surface of the recombinant immune cell, which can recognize HLA-G protein. After binding to HLA-G protein, the recombinant immune cell can achieve different types of signal outputs, such as activation of expression of specific genes, particularly activation of expression of a chimeric antigen receptor containing a factor having a therapeutic effect (e.g., for treating tumors), for use in treating diseases such as tumors.

It should be noted that the term "suitable conditions" as used herein refers to conditions suitable for the expression of the above chimeric polypeptide. It is readily understood by those skilled in the art that, conditions suitable for the expression of the above chimeric polypeptide include, but are not limited to, an appropriate transformation or transfection approach, an appropriate transformation or transfection condition, a healthy cell state, an appropriate cell density, a proper cell culture environment, and a suitable cell culture duration. The "suitable conditions" are not particularly limited. Those skilled in the art can optimize optimal conditions for the expression of the chimeric polypeptide based on a specific laboratory environment.

According to an embodiment of the present disclosure, the chimeric polypeptide in the recombinant immune cell is expressed by introducing the first expression vector described in the third aspect into a host cell.

According to an embodiment of the present disclosure, the recombinant immune cell further includes a chimeric antigen receptor. The chimeric antigen receptor is the chimeric antigen receptor encoded by the second nucleic acid fragment as defined in the second nucleic acid molecule described in the fourth aspect. Under suitable conditions, the recombinant immune cell according to an embodiment of the present disclosure can express both the above chimeric polypeptide and the chimeric antigen receptor from the above second nucleic acid molecule on the surface of the recombinant immune cell. Thus, the recombinant immune cell possesses multi-targeting capability and can recognize HLA-G protein. After binding to HLA-G protein, the recombinant immune cell can achieve different types of signal outputs, such as activation of expression of specific genes, particularly activation of expression of a chimeric antigen receptor containing a factor having a therapeutic effect (e.g., for treating tumors), for use in treating diseases such as tumors. In particular, the recombinant immune cell for treating tumors can be prepared, which can broadly recognize numerous tumor cells, significantly improving the efficiency and precision of killing various types of tumor cells. As a result, the problems of non-specific tumor recognition and incomplete tumor recognition coverage in current clinical applications of cell therapy are overcome, thereby providing a new tumor treatment method with broad application prospects.

In particular, when the chimeric polypeptide of the present disclosure is used in combination with a chimeric antigen receptor to jointly engineer and prepare recombinant immune cells (such as Jurkat cells and NK cells), it is found that immune cells co-expressing the chimeric polypeptide and CAR/TCR can recognize the corresponding ligand and efficiently activate the immune cells (such as Jurkat cells and NK cells) to perform a function of killing targets (e.g., tumor cells).

According to an embodiment of the present disclosure, the chimeric antigen receptor in the recombinant immune cell is expressed by introducing the second expression vector described in the fifth aspect into a host cell.

According to an embodiment of the present disclosure, the host cell includes at least one of an immune cell, a neuron, a progenitor cell or a precursor cell, an epithelial cell, an endothelial cell, or a stem cell.

According to an embodiment of the present disclosure, the host cell includes at least one of a T cell, a B cell, a monocyte, an NK cell, a dendritic cell, a macrophage, a regulatory T cell, a helper T cell, a cytotoxic T cell, an NKT cell, or a γδ T cell.

### Pharmaceutical Composition

In a seventh aspect of the present disclosure, the present disclosure provides a pharmaceutical composition. According to an embodiment of the present disclosure, the pharmaceutical composition includes the recombinant immune cell described in the sixth aspect. As previously mentioned, cells expressing the above chimeric polypeptide can recognize HLA-G protein. After binding to HLA-G protein, the cells can achieve different types of signal outputs, such as activation and suppression of expression of specific genes. Alternatively, after the contact with HLA-G protein, immune cells expressing the chimeric polypeptide can secrete a factor having a therapeutic effect for use in anti-tumor purposes. Therefore, the pharmaceutical composition including the above recombinant immune cell can be used to target HLA-G protein for prevention and/or treatment of a related disease, such as cancer.

According to an embodiment of the present disclosure, the pharmaceutical composition further includes a pharmaceutically acceptable excipient.

### Use

In an eighth aspect of the present disclosure, the present disclosure provides use of the recombinant immune cell described in the sixth aspect or the pharmaceutical composition described in the seventh aspect in the manufacture of a medicament for preventing and/or treating a disease.

According to an embodiment of the present disclosure, the disease includes cancer or a tumor, an autoimmune disease, inflammation, and a disease associated with cellular senescence.

As used herein, the terms "cancer" or "tumor" may refer to any uncontrolled cell growth. Exemplarily, the "cancer" or "tumor" may include small cell lung cancer, non-small cell lung cancer, papillary thyroid carcinoma, glioblastoma multiforme, colon cancer, rectal cancer, lung cancer, head and neck cancer, kidney cancer, bladder cancer, breast cancer, ovarian cancer, liver cancer, cholangiocarcinoma, sarcoma, acute myeloid leukemia, acute lymphoblastic leukemia, chronic myeloid leukemia, chronic lymphocytic leukemia, lymphoma, multiple myeloma, myelodysplastic syndromes, myeloproliferative neoplasms, large cell neuroendocrine carcinoma, neuroblastoma, prostate cancer, fibrosarcoma, pancreatic cancer, melanoma, head and neck squamous cell carcinoma, cervical cancer, skin cancer, glioma, esophageal cancer, nasopharyngeal carcinoma, oral squamous cell carcinoma, or gastric cancer.

### Method for Treating or Preventing Disease

In a ninth aspect of the present disclosure, the present disclosure provides a method for treating or preventing a disease. According to an embodiment of the present disclosure, the method includes administering to a subject a pharmaceutically acceptable amount of the recombinant immune cell described in the sixth aspect or the pharmaceutical composition described in the seventh aspect. As previously mentioned, cells expressing the above chimeric polypeptide can recognize HLA-G protein. After binding to HLA-G protein, the cells can achieve different types of signal outputs, such as activation and suppression of expression of specific genes. Alternatively, after the contact with HLA-G protein, immune cells expressing the chimeric polypeptide can secrete a factor having a therapeutic effect for use in anti-tumor purposes. Therefore, the pharmaceutical composition including the above recombinant immune cell can be used to target HLA-G protein for prevention and/or treatment of a related disease, such as cancer.

An effective amount of the recombinant protein or the pharmaceutical composition of the present disclosure may vary depending on the mode of administration, the severity of a disease to be treated, or the like. Selection of a preferred effective amount can be determined by those of ordinary skill in the art based on various factors (e.g., through clinical trials). These factors include, but are not limited to: a pharmacokinetic parameter of an active ingredient, such as bioavailability, metabolism, and half-life; severity of a disease to be treated in a patient, the body weight of a patient, the immune status of a patient, and the route of administration. For example, depending on the urgency of a therapeutic situation, several divided doses may be administered daily, or a dose may be proportionally reduced.

The recombinant protein or the pharmaceutical composition of the present disclosure may be incorporated into medicaments suitable for parenteral administration (e.g., intravenous, subcutaneous, intraperitoneal, and intramuscular). These medicaments may be formulated into various forms, such as a liquid form, a semi-solid form, and a solid dosage form, including but not limited to a liquid solution (e.g., an injectable solution and an infusible solution) or a lyophilized powder. A typical medicament is in the form of an injectable solution or an infusible solution. The above recombinant protein or pharmaceutical composition may be administered via intravenous infusion or injection, or via intramuscular or subcutaneous injection.

According to an embodiment of the present disclosure, the route of administration for the method is subcutaneous injection or intravenous injection.

According to an embodiment of the present disclosure, the disease includes cancer or tumor and an immune-related disease. According to an embodiment of the present disclosure, the disease includes cancer or a tumor, an autoimmune disease, inflammation, and a disease associated with cellular senescence.

According to an embodiment of the present disclosure, the tumor or cancer includes, but is not limited to, small cell lung cancer, non-small cell lung cancer, papillary thyroid carcinoma, glioblastoma multiforme, colon cancer, rectal cancer, lung cancer, head and neck cancer, kidney cancer, bladder cancer, breast cancer, ovarian cancer, liver cancer, cholangiocarcinoma, sarcoma, acute myeloid leukemia, acute lymphoblastic leukemia, chronic myeloid leukemia, chronic lymphocytic leukemia, lymphoma, multiple myeloma, myelodysplastic syndromes, myeloproliferative neoplasms, large cell neuroendocrine carcinoma, neuroblastoma, prostate cancer, fibrosarcoma, pancreatic cancer, melanoma, head and neck squamous cell carcinoma, cervical cancer, skin cancer, glioma, esophageal cancer, nasopharyngeal carcinoma, oral squamous cell carcinoma, or gastric cancer.

Solutions of the present disclosure are described below through examples. It should be understood by those skilled in the art that the examples described below are only used for illustrating the present disclosure and should not be construed as limiting the scope of the present disclosure. Where specific techniques or conditions are not indicated in the examples, the procedures shall be carried out in accordance with the techniques or conditions described in the literature in the field or in accordance with the product specification. The reagents or instruments used without the indication of the manufacturers are all conventional products that can be purchased commercially.

### Example 1: Design and Construction of SynNotch Receptor Specifically Targeting and Binding Human HLA-G

ILT2 and ILT4 receptors are two receptors on the surface of immune cells responsible for binding to HLA-G molecules. ILT4 receptor is expressed only on myeloid-derived immune cells, whereas ILT2 receptor is expressed on both lymphoid-derived and myeloid-derived immune cells. Both ILT2 and ILT4 receptors consist of an extracellular region, a transmembrane region, and an intracellular region. The extracellular region is a functional region responsible for recognizing and binding HLA-G. Further, the extracellular region of each of ILT2 and ILT4 receptors is composed of four domains, named Domain 1 (D1), Domain 2 (D2), Domain 3 (D3), and Domain 4 (D4).

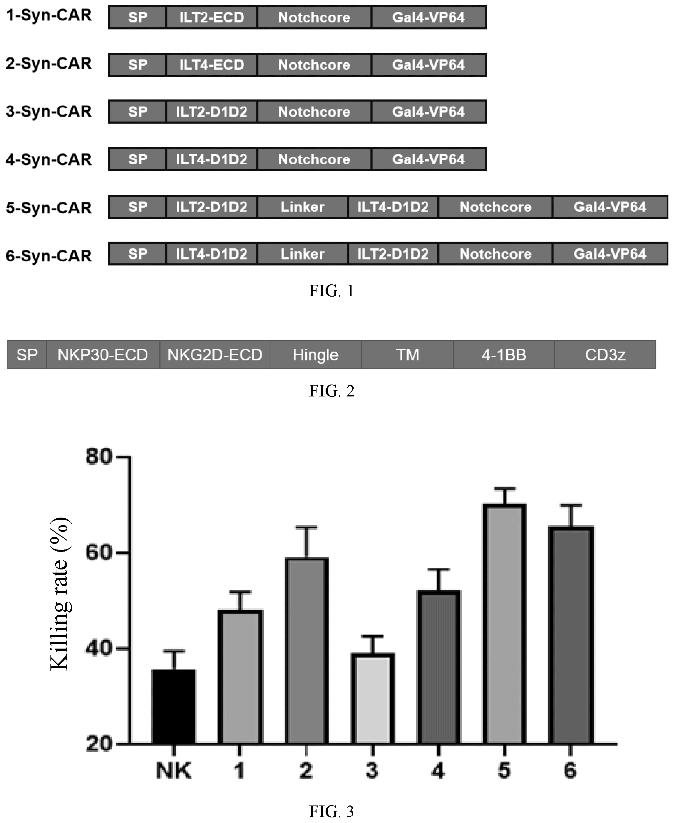

Based on the binding characteristic of ILT2 and ILT4 with HLA-G, a series of synthetic polypeptides capable of targeting and binding human HLA-G were designed and synthesized. These synthetic polypeptides were composed of polypeptide sequences derived from ILT2 and ILT4 receptors. Since the polypeptide sequences derived from both ILT2 and ILT4 possess an ability to bind HLA-G, the binding capability of the designed synthetic polypeptides to HLA-G was maximized. These synthetic polypeptides were used as the extracellular region of SynNotch receptor (the amino acid sequence of ILT2-ECD in 1-Syn-CAR is as set forth in SEQ ID NO: 6; the amino acid sequence of ILT4-ECD in 2-Syn-CAR is as set forth in SEQ ID NO: 11; the amino acid sequences of ILT2-D1D2 in 3-Syn-CAR are as set forth in SEQ ID NO: 2 and SEQ ID NO:3; the amino acid sequences of ILT4-D1D2 in 4-Syn-CAR are as set forth in SEQ ID NO: 7 and SEQ ID NO: 8; in 5-Syn-CAR, the amino acid sequences of ILT2-D1D2 are as set forth in SEQ ID NO: 2 and SEQ ID NO: 3, the amino acid sequence of Linker was GGGGS, and the amino acid sequences of ILT4-D1D2 are as set forth in SEQ ID NO: 7 and SEQ ID NO: 8; in 6-Syn-CAR, the amino acid sequences of ILT4-D1D2 are as set forth in SEQ ID NO: 7 and SEQ ID NO: 8, the amino acid sequence of Linker was GGGGS, and the amino acid sequences of ILT2-D1D2 are as set forth in SEQ ID NO: 2 and SEQ ID NO: 3). A CD8α-derived signal peptide sequence (amino acid sequence as set forth in SEQ ID NO: 19) was linked to their N-terminus. To their C-terminus, *Xenopus tropicalis* Notch transmembrane domain sequence (amino acid sequence as set forth in SEQ ID NO: 1) and Gal4-VP64 artificial transcription factor sequence (amino acid sequence as set forth in SEQ ID NO: 12) were sequentially linked, generating a series of SynNotch receptor sequences, as illustrated in FIG. 1. This series of sequences was individually cloned into pCDH vector. Viruses were produced, and used to infect NK cells or Jurkat cells. A stable transgenic cell line was established for each sequence.

### Example 2: Design and Construction of CAR Receptor Specifically Targeting and Binding Human NKG2D Ligand and NKp30 Ligand

NKG2D and NKp30 receptors are two receptors on the surface of immune cells responsible for binding molecular ligands such as MICA/B, ULBPs, and B7H6. Ligands for NKG2D and NKp30 are widely expressed on the surface of many tumor cells and are not expressed or expressed at low levels in most normal tissues. Both NKG2D and NKp30 receptors consist of an extracellular region, a transmembrane region, and an intracellular region. The extracellular region is a functional region responsible for recognizing and binding the corresponding ligand.

Based on the binding characteristic of NKG2D and NKp30 with the above ligands, synthetic polypeptides capable of targeting and binding human NKG2D ligand and NKp30 ligand were designed and synthesized. These synthetic polypeptides were composed of extracellular polypeptide sequences from both NKp30 and NKG2D, enabling binding to ligands for both NKG2D and NKp30. These synthetic polypeptides were used as the extracellular region of a CAR receptor (the C-terminus of NKp30 receptor extracellular region is linked to the N-terminus of NKG2D receptor extracellular region; the amino acid sequence of NKp30 receptor extracellular region is as set forth in SEQ ID NO: 14, and the amino acid sequence of NKG2D receptor extracellular region is as set forth in SEQ ID NO: 13). A CD8α-derived signal peptide sequence (amino acid sequence as set forth in SEQ ID NO: 19 and nucleotide sequence as set forth in SEQ ID NO: 20) was linked to their N-terminus. To their C-terminus, a CD8α-derived hinge region sequence (amino acid sequence as set forth in SEQ ID NO: 15), a CD8α-derived transmembrane domain sequence (amino acid sequence as set forth in SEQ ID NO: 16), a 4-1BB sequence, and a CD3ζ sequence (the amino acid sequence of 4-1BB and CD3ζ as set forth in SEQ ID NO: 17) were sequentially linked, generating a CAR receptor sequence. Additionally, a UAS-mini-CMV sequence (nucleotide sequence as set forth in SEQ ID NO: 21) was added upstream of the nucleotide sequence encoding the CAR receptor, as illustrated in FIG. 2. This sequence was cloned into a promoter-removed pCDH vector to produce virus. The virus was used to infect the stable transgenic NK cells established in Example 1, creating stable cell lines transfected with two receptor nucleic acid sequences, named the corresponding Syn-CAR-NK cell line and Syn-CAR-Jurkat cell line. Further, the CAR receptor without the UAS-mini-CMV sequence was cloned into a pCDH vector containing a promoter to produce virus. The virus was used to infect NK and Jurkat cells to establish stable transgenic cell lines, which served as controls for Syn-CAR-NK/Jurkat cells, and were named CAR-NK cells and CAR-Jurkat cells.

### Example 3: In Vitro Experimental Validation of the Precise and Efficient Target Cell Killing by Syn-CAR-NK Cells

The *in vitro* killing activity of different types of Syn-CAR-NK cells obtained in Example 2 was tested via PI and CFSE staining. Specifically, for the tumor cell line K562 (which possesses all targets required for Syn-CAR-NK activation), this target cell line was fluorescently stained with CFSE and seeded into culture plates at a concentration of 2×10⁴ cells/ml per well. Six experimental groups and one control group were set up correspondingly for the target cell line. The experimental groups received a cell suspension of Syn-CAR-NK cells obtained in Example 2, which targeted all of HLA-G, NKG2D ligand, and NKp30 ligand. The blank control group received NK cells infected with empty vector virus. In the above experimental groups, Syn-CAR-NK cells were mixed with target cells at an effector-to-target ratio (the term "effector-to-target ratio" herein refers to the numerical ratio of effector cells, i.e., Syn-CAR-NK cells targeting all of HLA-G, NKG2D ligand, and NKp30 ligand, to target cells, i.e., tumor cells) of 1:2 for 48 hours. After 48 hours of co-culture, the supernatant was removed by centrifugation. The cell pellet was washed and then stained with PI. Stained cells were obtained using a flow cytometer and the results were analyzed using software FlowJo. The results of tumor cell killing rate using leukemia cell line K562 as target cells are shown in FIG. 3. It can be seen from FIG. 3 that Syn-CAR-NK cells exhibited a significant killing effect on leukemia cells K562 (markedly higher than that of the control group), with 5-Syn-CAR-NK and 6-Syn-CAR-NK demonstrating the highest killing efficiency.

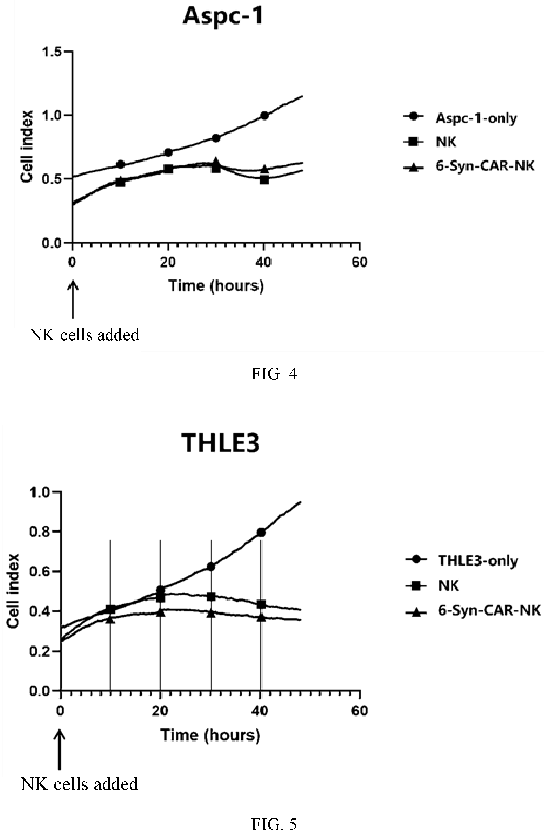

For tumor cell line Aspc-1 (possessing only a target activating SynNotch) and immortalized normal cell line THLE3 (lacking any target for activating either SynNotch or CAR), after 5×10³ target cells were adhered to an E-plate, cells from the experimental groups and the control groups in Example 2 were added. The growth status of target cells was subsequently monitored using an RTCA instrument. The results show that cells in the experimental group of the present disclosure exhibited no significant killing effect on pancreatic cancer cells Aspc-1 that were negative for both NKG2D ligand and NKp30 ligand (similar to the killing effect observed in the control group). In this example, the results for 6-Syn-CAR-NK are illustratively presented. The results of tumor cell killing rate using pancreatic cancer Aspc-1 as target cells are shown in FIG. 4. The results of killing rate using the immortalized liver cell line as target cells are shown in FIG. 5.

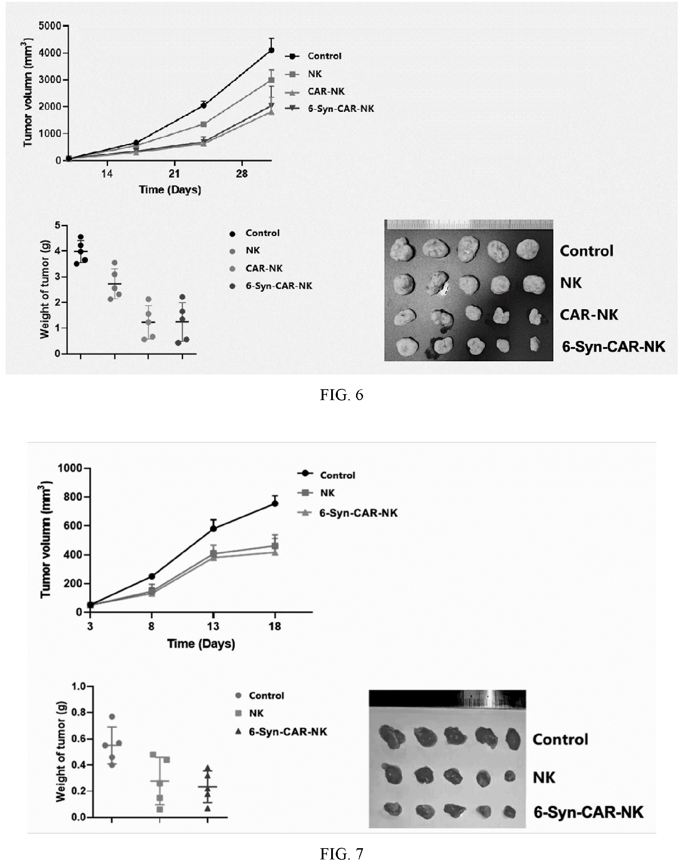

It can be seen from FIG. 4 that 6-Syn-CAR-NK cells exhibited no significant killing effect on pancreatic cancer cells Aspc-1 that were negative for both NKG2D ligand and NKp30 ligand (similar to the killing effect observed in the control group). As shown in FIG. 5, 6-Syn-CAR-NK cells exhibited no significant enhancement in killing effect on immortalized liver cells THLE3 that were negative for HLA-G, NKG2D ligand, and NKp30 ligand (similar to the killing effect observed in the control group). These results indicate that the Syn-CAR system functions effectively, and exhibits no killing effect on cells expressing only a single target or lacking target expression, enabling a more precise distinction between normal cells and tumor cells.

### Example 4: In Vivo Experimental Validation of the Precise and Efficient Target Cell Killing by Syn-CAR-NK Cells

1. 1×10⁷ NCI-H716 cells were subcutaneously injected into 5-week-old NCG mice. Ten days after tumor inoculation, tumor sizes were measured using a vernier caliper. Mice with similar tumor burdens were randomly divided into nine groups. Six of the nine groups served as experimental groups (receiving different types of Syn-CAR-NK cell obtained in Example 2), while the remaining three groups served as control groups (one as a no-treatment control, one as a regular NK treatment control, and one as a CAR-NK treatment control). Subsequently, 1×10⁷ 6-Syn-CAR-NK cells were injected via the tail vein into the mice in the experimental groups. 1×10⁷ regular NK cells were injected via the tail vein into the mice in one control group. 1×10⁷ CAR-NK cells were injected via the tail vein into the mice in another control group. An equal volume of saline was injected via the tail vein into the mice in the remaining control group. Thereafter, each group received the same treatment once per week, and sizes of the subcutaneous xenograft tumors were measured. During this period, IL2 was intraperitoneally injected every three days at a dose of 5×10⁴ U per mouse per injection. A total of three treatments were performed. The results reveal that Syn-CAR-NK cells of the present disclosure exhibited a significant killing effect on the target cells, i.e., colon cancer cells NCI-H716 that co-expressed HLA-G, NKG2D ligand, and NKp30 ligand (significantly higher than that of the NK control group), and their killing efficiency showed no significant difference compared with the CAR-NK-only group. In this example, the results for 6-Syn-CAR-NK are exemplarily presented. Reference can be made to FIG. 6 for details (test results of killing rate of colon cancer cells NCI-H716 by 6-Syn-CAR-NK cells after tumor inoculation).

It can be seen from FIG. 6 that 6-Syn-CAR-NK cells of Example 4 exhibited a significant *in vivo* killing effect on the target cells, i.e., colon cancer cells NCI-H716 that co-expressed HLA-G, NKG2D ligand, and NKp30 ligand (significantly higher than that of the NK control group), and their killing efficiency showed no significant difference compared with the CAR-NK-only group, indicating that the SynNotch receptor did not reduce their killing efficiency.

2. Further, 1×10⁷ Aspc-1 cells were subcutaneously injected into 5-week-old NCG mice. Ten days after tumor inoculation, tumor sizes were measured using a vernier caliper. Mice with similar tumor burdens were randomly divided into three groups: one experimental group treated with 6-Syn-CAR-NK cells and two control groups (one as a no-treatment control and one as a regular NK treatment control). Subsequently, 1×10⁷ 6-Syn-CAR-NK cells were injected via the tail vein into the mice in the experimental group. 1×10⁷ regular NK cells were injected via the tail vein into the mice in one control group. An equal volume of saline was injected via the tail vein into the mice in the remaining control group. Thereafter, each group received the same treatment once per week, and sizes of the subcutaneous xenograft tumors were measured. During this period, IL2 was intraperitoneally injected every three days at a dose of 5×10⁴ U per mouse per injection. A total of three treatments were performed. The results of killing rate of pancreatic cancer cells Aspc-1 by 6-Syn-CAR-NK cells after tumor inoculation are shown in FIG. 7.

It can be seen from FIG. 7 that 6-Syn-CAR-NK cells from Example 4 exhibited no significant enhancement in the *in vivo* killing effect on the target cells that expressed only HLA-G, i.e., pancreatic cancer cells Aspc-1 (no increase in the killing efficiency was observed compared with the NK control group), which demonstrates that the Syn-CAR system functions effectively, and exhibits no killing effect on cells expressing only a single target, enabling a more precise distinction between normal cells and tumor cells.

### Example 5: In vitro Experimental Validation of the Precise and Efficient Target Cell Killing by Syn-CAR-Jurkat cells

The *in vitro* killing activity of different types of 6-Syn-CAR-Jurkat cells obtained in Example 2 was assessed by flow cytometry. Specifically, for the tumor cell line K562 (which possesses all targets required for Syn-CAR-Jurkat activation), this target cell line was seeded into culture plates at a concentration of 2×10⁴ cells/ml per well. For this target cell line, one experimental group and one control group were established. The experimental group received a cell suspension of 6-Syn-CAR-Jurkat cells obtained in Example 2, which targeted all of HLA-G, NKG2D ligand, and NKp30 ligand. The blank control group received Jurkat cells infected with empty vector virus. In the above experimental group, 6-Syn-CAR-Jurkat cells were mixed with target cells at an effector-to-target ratio (the term "effector-to-target ratio" herein refers to a numerical ratio of effector cells, i.e., 6-Syn-CAR-Jurkat cells targeting all of HLA-G, NKG2D ligand, and NKp30 ligand, to target cells, i.e., tumor cells) of 1:2 for 48 hours. After 48 hours of co-culture, the supernatant was removed by centrifugation. The cell pellet was washed and then stained with antibodies against TNF-α, IFN-γ, and CD69. Stained cells were obtained using a flow cytometer, and the results were analyzed using software FlowJo to evaluate the activation effect of Jurkat cells using leukemia cells K562 as target cells. The results show that 6-Syn-CAR-Jurkat cells were significantly activated by leukemia cells K562, with markedly increased expression of TNF-α, IFN-γ, and CD69 (significantly higher than that of the control group), demonstrating that the Syn-CAR system designed in the present disclosure also functions effectively in a CAR-T cell platform.

Throughout this specification, description with reference to "an embodiment", "some embodiments", "an example", "a specific example", or "some examples", means that a particular feature, structure, material, or characteristic described in connection with the embodiment or example is included in at least one embodiment or example of the present disclosure. The appearances of the above phrases in various places throughout this specification are not necessarily referring to the same embodiment or example of the present disclosure. Further, the particular features, structures, materials, or characteristics described here may be combined in any suitable manner in one or more embodiments or examples. In addition, different embodiments or examples and features of different embodiments or examples described in the specification may be combined by those skilled in the art without mutual contradiction.

Although embodiments of the present disclosure have been shown and described above, it should be understood that the above embodiments are merely exemplary, and cannot be construed to limit the present disclosure. For those skilled in the art, changes, alternatives, and modifications can be made to the embodiments without departing from the scope of the present disclosure.

## Claims

1. A chimeric polypeptide, comprising:
a first extracellular region, having an activity of binding to HLA-G protein;
a first transmembrane region, comprising a transmembrane region of *Xenopus tropicalis* Notch receptor protein or an amino acid sequence having at least 80% identity thereto, the N-terminus of the first transmembrane region being linked to the C-terminus of the first extracellular region; and
a first intracellular region, the N-terminus of the first intracellular region being linked to the C-terminus of the first transmembrane region.

2. The chimeric polypeptide according to claim 1, wherein the transmembrane region of the *Xenopus tropicalis* Notch receptor protein has the amino acid sequence as set forth in SEQ ID NO: 1;
optionally, the transmembrane region further comprises an epidermal growth factor-like repeat sequence and/or an RAM sequence;
optionally, the C-terminus of the epidermal growth factor-like repeat sequence is linked to the N-terminus of the transmembrane region of the *Xenopus tropicalis* Notch receptor protein, and/or the C-terminus of the transmembrane region of the *Xenopus tropicalis* Notch receptor protein is linked to the N-terminus of the RAM sequence;
optionally, the first transmembrane region has the amino acid sequence as set forth in SEQ ID NO: 1 or SEQ ID NO: 25, or an amino acid sequence having at least 90% identity thereto;
optionally, the first transmembrane region has the amino acid sequence as set forth in SEQ ID NO: 1 or SEQ ID NO: 25.

3. The chimeric polypeptide according to claim 1, wherein the first extracellular region comprises a first binding protein or fragment thereof for binding to HLA-G protein;
optionally, the first binding protein or fragment thereof comprises at least one of an antibody or functional fragment thereof, or a receptor;
optionally, the first binding protein or fragment thereof comprises a first binding moiety and/or a second binding moiety, wherein:
the first binding moiety is an extracellular region of ILT2 protein or active fragment thereof, and
the second binding moiety is an extracellular region of ILT4 protein or active fragment thereof;
optionally, the first binding protein or fragment thereof comprises the first binding moiety and the second binding moiety, the second binding moiety being linked to the first binding moiety;
optionally, the extracellular region of ILT2 protein is composed of ILT2-D1 domain, ILT2-D2 domain, ILT2-D3 domain, and ILT2-D4 domain, wherein:
the ILT2-D1 domain has the amino acid sequence as set forth in SEQ ID NO: 2;
the ILT2-D2 domain has the amino acid sequence as set forth in SEQ ID NO: 3;
the ILT2-D3 domain has the amino acid sequence as set forth in SEQ ID NO: 4; and
the ILT2-D4 domain has the amino acid sequence as set forth in SEQ ID NO: 5;
optionally, the extracellular region of ILT2 protein has the amino acid sequence as set forth in SEQ ID NO: 6;
optionally, the first binding moiety is selected from:
at least one of the ILT2-D1 domain, the ILT2-D2 domain, the ILT2-D3 domain, or the ILT2-D4 domain, or
the extracellular region of ILT2 protein;
optionally, the first binding moiety comprises the ILT2-D1 domain and the ILT2-D2 domain, the ILT2-D1 domain being linked to the ILT2-D2 domain;
optionally, the C-terminus of the ILT2-D1 domain is linked to the N-terminus of the ILT2-D2 domain, or the N-terminus of the ILT2-D1 domain is linked to the C-terminus of the ILT2-D2 domain;
optionally, the first binding moiety is the extracellular region of ILT2 protein;
optionally, the extracellular region of ILT4 protein is composed of ILT4-D1 domain, ILT4-D2 domain, ILT4-D3 domain, and ILT4-D4 domain, wherein:
the ILT4-D1 domain has the amino acid sequence as set forth in SEQ ID NO: 7;
the ILT4-D2 domain has the amino acid sequence as set forth in SEQ ID NO: 8;
the ILT4-D3 domain has the amino acid sequence as set forth in SEQ ID NO: 9; and
the ILT4-D4 domain has the amino acid sequence as set forth in SEQ ID NO: 10;
optionally, the extracellular region of ILT4 protein has the amino acid sequence as set forth in SEQ ID NO: 11;
optionally, the second binding moiety is selected from at least one of the ILT4-D1 domain, the ILT4-D2 domain, the ILT4-D3 domain, or the ILT4-D4 domain, or the extracellular region of ILT4 protein;
optionally, the second binding moiety comprises the ILT4-D1 domain and the ILT4-D2 domain, the ILT4-D1 domain being linked to the ILT4-D2 domain;
optionally, the C-terminus of the ILT4-D1 domain is linked to the N-terminus of the ILT4-D2 domain, or the N-terminus of the ILT4-D1 domain is linked to the C-terminus of the ILT4-D2 domain;
optionally, the second binding moiety is the extracellular region of ILT4 protein;
optionally, when the first extracellular region comprises a plurality of the first binding protein or fragment thereof, the first extracellular region further comprises a first linker peptide, any two of the plurality of the first binding protein or fragment thereof being linked with or without the first linker peptide;
optionally, the first binding protein or fragment thereof comprises the first binding moiety and the second binding moiety, the first binding moiety and the second binding moiety being linked via the first linker peptide;
optionally, the C-terminus of the first binding moiety is linked to the N-terminus of the first linker peptide and the C-terminus of the first linker peptide is linked to the N-terminus of the second binding moiety, or the C-terminus of the second binding moiety is linked to the N-terminus of the first linker peptide and the C-terminus of the first linker peptide is linked to the N-terminus of the first binding moiety;
optionally, the amino acid sequence of the first linker peptide is (GGGGS)n, where n is any integer ranging from 1 to 10;
optionally, n is 1, 2, 3 or 4;
optionally, the amino acid sequence of the first linker peptide is GGGGS;
optionally, the first extracellular region is selected from: ILT2-D1 domain + ILT2-D2 domain + first linker peptide + ILT4-D1 domain + ILT4-D2 domain; ILT4-D1 domain + ILT4-D2 domain + first linker peptide + ILT2-D1 domain + ILT2-D2 domain; the extracellular region of ILT2 protein + first linker peptide + the extracellular region of ILT4 protein; the extracellular region of ILT4 protein + first linker peptide + the extracellular region of ILT2 protein; ILT2-D1 domain + ILT2-D2 domain + first linker peptide + the extracellular region of ILT4 protein;; ILT4-D1 domain + ILT4-D2 domain + first linker peptide + the extracellular region of ILT2 protein; ILT2-D1 domain + first linker peptide + ILT4-D1 domain; ILT2-D1 domain + first linker peptide + ILT4-D2 domain; ILT2-D1 domain + first linker peptide + ILT4-D1 domain + ILT4-D2 domain; ILT2-D1 domain + first linker peptide + the extracellular region of ILT4 protein; ILT2-D1 domain + first linker peptide + ILT2-D1 domain; ILT2-D1 domain + first linker peptide + ILT2-D2 domain; ILT2-D1 domain + first linker peptide + ILT2-D1 domain + ILT2-D2 domain; ILT2-D1 domain + first linker peptide + the extracellular region of ILT2 protein; ILT4-D1 domain + first linker peptide + ILT4-D1 domain; ILT4-D1 domain + first linker peptide + ILT4-D2 domain; ILT4-D1 domain + first linker peptide + ILT4-D1 domain + ILT4-D2 domain; ILT4-D1 domain + first linker peptide + the extracellular region of ILT4 protein; ILT4-D1 domain + first linker peptide + ILT2-D1 domain; ILT4-D1 domain + first linker peptide + ILT2-D2 domain; ILT4-D1 domain + first linker peptide + ILT2-D1 domain + ILT2-D2 domain; ILT4-D1 domain + first linker peptide + the extracellular region of ILT2 protein; ILT2-D2 domain + first linker peptide + ILT4-D1 domain; ILT2-D2 domain + first linker peptide + ILT4-D2 domain; ILT2-D2 domain + first linker peptide + ILT4-D1 domain + ILT4-D2 domain; ILT2-D2 domain + first linker peptide + the extracellular region of ILT4 protein; ILT2-D2 domain + first linker peptide + ILT2-D1 domain; ILT2-D2 domain + first linker peptide + ILT2-D2 domain; ILT2-D2 domain + first linker peptide + ILT2-D1 domain + ILT2-D2 domain; ILT2-D2 domain + first linker peptide + the extracellular region of ILT2 protein; ILT4-D2 domain + first linker peptide + ILT4-D1 domain; ILT4-D2 domain + first linker peptide + ILT4-D2 domain; ILT4-D2 domain + first linker peptide + ILT4-D1 domain + ILT4-D2 domain; ILT4-D2 domain + first linker peptide + the extracellular region of ILT4 protein; ILT4-D2 domain + first linker peptide + ILT2-D1 domain; ILT4-D2 domain + first linker peptide + ILT2-D2 domain; ILT4-D2 domain + first linker peptide + ILT2-D1 domain + ILT2-D2 domain; ILT4-D2 domain + first linker peptide + the extracellular region of ILT2 protein; ILT2-D1 domain + ILT2-D2 domain + first linker peptide + ILT4-D1 domain; ILT2-D1 domain + ILT2-D2 domain + first linker peptide + ILT4-D2 domain; ILT2-D1 domain + ILT2-D2 domain + first linker peptide + ILT4-D1 domain + ILT4-D2 domain; ILT2-D1 domain + ILT2-D2 domain + first linker peptide + the extracellular region of ILT4 protein; ILT2-D1 domain + ILT2-D2 domain + first linker peptide + ILT2-D1 domain; ILT2-D1 domain + ILT2-D2 domain + first linker peptide + ILT2-D2 domain; ILT2-D1 domain + ILT2-D2 domain + first linker peptide + ILT2-D1 domain + ILT2-D2 domain; ILT2-D1 domain + ILT2-D2 domain + first linker peptide + the extracellular region of ILT2 protein; ILT4-D1 domain + ILT4-D2 domain + first linker peptide + ILT4-D1 domain; ILT4-D1 domain + ILT4-D2 domain + first linker peptide + ILT4-D2 domain; ILT4-D1 domain + ILT4-D2 domain + first linker peptide + ILT4-D1 domain + ILT4-D2 domain; ILT4-D1 domain + ILT4-D2 domain + first linker peptide + the extracellular region of ILT4 protein; ILT4-D1 domain + ILT4-D2 domain + first linker peptide + ILT2-D1 domain; ILT4-D1 domain + ILT4-D2 domain + first linker peptide + ILT2-D2 domain; ILT4-D1 domain + ILT4-D2 domain + first linker peptide + ILT2-D1 domain + ILT2-D2 domain; ILT4-D1 domain + ILT4-D2 domain + first linker peptide + the extracellular region of ILT2 protein; the extracellular region of ILT2 protein + first linker peptide + ILT4-D1 domain; the extracellular region of ILT2 protein + first linker peptide + ILT4-D2 domain; the extracellular region of ILT2 protein + first linker peptide + ILT4-D1 domain + ILT4-D2 domain; the extracellular region of ILT2 protein + first linker peptide + the extracellular region of ILT4 protein; the extracellular region of ILT2 protein + first linker peptide + ILT2-D1 domain; the extracellular region of ILT2 protein + first linker peptide + ILT2-D2 domain; the extracellular region of ILT2 protein + first linker peptide + ILT2-D1 domain + ILT2-D2 domain; the extracellular region of ILT2 protein + first linker peptide + the extracellular region of ILT2 protein; the extracellular region of ILT4 protein + first linker peptide + ILT4-D1 domain; the extracellular region of ILT4 protein + first linker peptide + ILT4-D2 domain; the extracellular region of ILT4 protein + first linker peptide + ILT4-D1 domain + ILT4-D2 domain; the extracellular region of ILT4 protein + first linker peptide + the extracellular region of ILT4 protein; the extracellular region of ILT4 protein + first linker peptide + ILT2-D1 domain; the extracellular region of ILT4 protein + first linker peptide + ILT2-D2 domain; the extracellular region of ILT4 protein + first linker peptide + ILT2-D1 domain + ILT2-D2 domain; or the extracellular region of ILT4 protein + first linker peptide + the extracellular region of ILT2 protein;
optionally, the first extracellular region is selected from:
1) the extracellular region of ILT2 protein;
2) the extracellular region of ILT4 protein;
3) ILT2-D1 domain and ILT2-D2 domain, from the N-terminus to the C-terminus;
4) ILT4-D1 domain and ILT4-D2 domain, from the N-terminus to the C-terminus;
5) ILT2-D1 domain, ILT2-D2 domain, the first linker peptide, ILT4-D1 domain, and ILT4-D2 domain, from the N-terminus to the C-terminus; or
6) ILT4-D1 domain, ILT4-D2 domain, the first linker peptide, ILT2-D1 domain, and ILT2-D2 domain, from the N-terminus to the C-terminus.

4. The chimeric polypeptide according to claim 1, wherein the first intracellular region comprises at least one of a transcriptional activator, a transcriptional repressor, a transcription factor, a site-specific nuclease, a recombinase, an activating immune receptor intracellular domain, or an inhibitory immune receptor intracellular domain;
optionally, the first intracellular region comprises at least one of GaL4-VP64, GaL4-VP16, tetR-VP64, ZFHD1-VP64, Gal4-KRAB, HAP1-VP16, or LexA-VP64;
optionally, Gal4-VP64 has the amino acid sequence as set forth in SEQ ID NO: 12.

5. A first nucleic acid molecule, encoding the chimeric polypeptide according to any one of claims 1 to 4;
optionally, the first nucleic acid molecule is DNA.

6. A first expression vector, carrying the first nucleic acid molecule according to claim 5; optionally, the first expression vector is a eukaryotic expression vector, a prokaryotic expression vector, a virus, or a bacteriophage;
preferably, the first expression vector is a plasmid expression vector.

7. A second nucleic acid molecule, comprising a first nucleic acid fragment and a second nucleic acid fragment, the 3' end of the first nucleic acid fragment being linked to the 5' end of the second nucleic acid fragment,
wherein the second nucleic acid fragment is used to encode a chimeric antigen receptor targeting a first molecule, and wherein the first nucleic acid fragment is used to bind to a first intracellular region and induce expression of the chimeric antigen receptor;
wherein the first intracellular region is the first intracellular region as defined in the chimeric polypeptide according to any one of claims 1 to 4.

8. The second nucleic acid molecule according to claim 7, wherein the chimeric antigen receptor comprises:
a second extracellular region, having a binding activity to the first molecule, wherein the first molecule is not HLA-G protein;
a second transmembrane region, the N-terminus of the second transmembrane region being linked to the C-terminus of the second extracellular region; and
a second intracellular region, the N-terminus of the second intracellular region being linked to the C-terminus of the second transmembrane region,
optionally, the first molecule comprises at least one of a tumor antigen, a virus, a bacterium, an endotoxin, an antibody, a cell receptor, or a ligand of a cell receptor;
optionally, the tumor antigen comprises at least one of a tumor-associated antigen or a tumor-specific antigen, preferably a tumor-specific antigen;
optionally, the first molecule comprises at least one of MICA, MICB, ULBPs, B7H6, B7H3, GFP, eGFP, CD19, ALPPL2, BCMA, SIRPα, CD1a, CD1b, CD1c, CD1d, CD1e, CD2, CD3d, CD3e, CD3g, CD4, CD5, CD7, CD8a, CD8b, CD20, CD21, CD22, CD23, CD25, CD27, CD28, CD30, CD33, CD34, CD38, CD40, CD44, CD44v6, CD45, CD48, CD51, CD52, CD56, CD59, CD66, CD70, CD71, CD72, CD73, CD74, CD79A, CD79B, CD80, CD86, CD94, CD95, CD123, CD133, CD134, CD140, CD152, CD154, CD158, CD178, CD181, CD182, CD183, CD200, CD210, CD221, CD246, CD252, CD253, CD261, CD262, CD269, CD273, CD274, CD276, CD279, CD295, CD339, CD340, EGFR, EGFR VIII, HER2, FGFR2, AFP, CA125, MSLN, GPC3, CEA, CLDN1, CLDN3, CLDN6, CLDN18.1, CLDN18.2, EpCAM, PSCA, GD2, GD3, IL-13, IL-13RA2, ROR1, MUC-1, PSMA, MAGEA1, 4-1BB, 5T4, BAFF, CA242, CA-IX, MET, CCR4, CNTO888, FAP, MORAb-009, EPHA2, VEGF-A, VEGFR-1, or VEGFR-2;
optionally, the second extracellular region comprises a second binding protein or fragment thereof for binding to the first molecule;
optionally, the second binding protein or fragment thereof comprises at least one of an antibody or functional fragment thereof, a receptor, a receptor ligand, or a cell adhesion molecule;
optionally, the second binding protein or fragment thereof is an extracellular region of an activating receptor expressed on the surface of an immune cell, or an antibody or a fragment thereof binding to the first molecule;
optionally, the antibody or fragment thereof binding to the first molecule is a single-chain antibody;
optionally, the activating receptor is a receptor expressed on the surface of NK cells;
optionally, the activating receptor is selected from at least one of NKG2D, NKp30, NKp44, NKp46, DNAM-1, PD-1, TIGIT, NKG2A, NKG2B, NKG2C, NKG2E, NKG2H, CD16, NKp80, CD226, CD160, CD161, CD96, PVRIG, SLAM, CD200R, CD49a, TIM-3, LAG-3, CD112R, KIR2DS1, KIR2DS2, KIR2DS4, KIR2DL1, KIR2DL2, KIR2DL3, KIR3DL1, KIR3DL2, LIR1, LIR2, SIGLE3, SIGLE7, SIGLE9, or KLRG1;
optionally, the activating receptor is selected from NKG2D and/or NKp30;
optionally, the second binding protein or fragment thereof comprises a third binding moiety and/or a fourth binding moiety, wherein:
the third binding moiety is an extracellular region of NKG2D or active fragment thereof; and
the fourth binding moiety is an extracellular region of NKp30 or active fragment thereof;
optionally, the extracellular region of NKG2D has the amino acid sequence as set forth in SEQ ID NO: 13;
optionally, the extracellular region of NKp30 has the amino acid sequence as set forth in SEQ ID NO: 14;
optionally, the third binding moiety has the amino acid sequence as set forth in SEQ ID NO: 13 or an amino acid sequence having at least 90% identity thereto;
optionally, the fourth binding moiety has the amino acid sequence as set forth in SEQ ID NO: 14 or an amino acid sequence having at least 90% identity thereto;
optionally, the second extracellular region further comprises a second linker peptide, the third binding moiety and the fourth binding moiety being linked via the second linker peptide;
optionally, the C-terminus of the third binding moiety is linked to the N-terminus of the second linker peptide and the C-terminus of the second linker peptide is linked to the N-terminus of the fourth binding moiety, or the C-terminus of the fourth binding moiety is linked to the N-terminus of the second linker peptide and the C-terminus of the second linker peptide is linked to the N-terminus of the third binding moiety;
optionally, the amino acid sequence of the second linker peptide is (GGGGS)n, where n is any integer ranging from 0 to 10;
optionally, n is 0, 1, 2, 3, or 4;
optionally, the amino acid sequence of the second linker peptide is GGGGS;
optionally, the second extracellular region further comprises a hinge region, wherein:
the C-terminus of the third binding moiety is linked to the N-terminus of the second linker peptide, the C-terminus of the second linker peptide is linked to the N-terminus of the fourth binding moiety, and the C-terminus of the fourth binding moiety is linked to the N-terminus of the hinge region, or
the C-terminus of the fourth binding moiety is linked to the N-terminus of the second linker peptide, the C-terminus of the second linker peptide is linked to the N-terminus of the third binding moiety, and the C-terminus of the third binding moiety is linked to the N-terminus of the hinge region;
optionally, the hinge region comprises at least one of a hinge region of CD8α molecule or variant thereof, or a hinge region of an immunoglobulin or variant thereof;
optionally, the hinge region has the amino acid sequence as set forth in SEQ ID NO: 15.

9. The second nucleic acid molecule according to claim 8, wherein the second transmembrane region is selected from at least one of a transmembrane region of CD8α molecule, a transmembrane region of CD28 molecule, a transmembrane region of CD3ζ molecule, a transmembrane region a CD4 molecule, a transmembrane region of CD16 molecule, a transmembrane region of 4-1BB molecule, a transmembrane region of OX40 molecule, a transmembrane region of ICOS molecule, a transmembrane region of CTLA-4 molecule, a transmembrane region of PD-1 molecule, a transmembrane region of LAG-3 molecule, a transmembrane region of 2B4 molecule, a transmembrane region of NKG2D molecule, a transmembrane region of DNAM-1 molecule, a transmembrane region of NKp44 molecule, a transmembrane region of NKp46 molecule, a transmembrane region of KIR2DS1 molecule, a transmembrane region of KIR2DS2 molecule, a transmembrane region of KIR2DS4 molecule, or a transmembrane region of BTLA molecule;
optionally, the second transmembrane region is the transmembrane region of CD8α molecule;
optionally, the second transmembrane region has the amino acid sequence as set forth in SEQ ID NO: 16;
optionally, the second intracellular region comprises an intracellular signaling domain and a co-stimulatory domain;
optionally, the intracellular signaling domain is selected from at least one of an intracellular signaling domain of CD3ζ molecule or an intracellular signaling domain of FcεRIγ molecule;
optionally, the co-stimulatory domain is selected from at least one of an intracellular signaling domain of 4-1BB molecule, an intracellular signaling domain of CD28 molecule, an intracellular signaling domain of CD27 molecule, an intracellular signaling domain of CD40 molecule, an intracellular signaling domain of OX40 molecule, an intracellular signaling domain of ICOS molecule, an intracellular signaling domain of DAP10 molecule, an intracellular signaling domain of DAP12 molecule, or an intracellular signaling domain of DNAM-1 molecule;
optionally, the second intracellular region has the amino acid sequence as set forth in SEQ ID NO: 17;
optionally, the second nucleic acid fragment has the nucleotide sequence as set forth in SEQ ID NO: 18.

10. The second nucleic acid molecule according to any one of claims 7 to 9, wherein the second nucleic acid molecule further comprises a third nucleic acid fragment encoding a signal peptide, wherein:
the 3' end of the first nucleic acid fragment is linked to the 5' end of the third nucleic acid fragment, and the 3' end of the third nucleic acid fragment is linked to the 5' end of the second nucleic acid fragment;
optionally, the signal peptide is selected from at least one of a signal peptide of CD8α molecule, a signal peptide of an IgG molecule, or a signal peptide of CD28 molecule;
optionally, the signal peptide has the amino acid sequence as set forth in SEQ ID NO: 19;
optionally, the third nucleic acid fragment has the nucleotide sequence as set forth in SEQ ID NO: 20;
optionally, the first nucleic acid fragment has the nucleotide sequence as set forth in SEQ ID NO: 21.

11. A second expression vector, carrying the second nucleic acid molecule according to any one of claims 7 to 10;
optionally, the second expression vector is a eukaryotic expression vector, a prokaryotic expression vector, a virus, or a bacteriophage;
preferably, the second expression vector is a plasmid expression vector.

12. A recombinant immune cell, carrying the first nucleic acid molecule according to claim 5 or the first expression vector according to claim 6; or expressing the chimeric polypeptide according to any one of claims 1 to 4.

13. The recombinant immune cell according to claim 12, wherein the chimeric polypeptide in the recombinant immune cell is expressed by introducing the first expression vector according to claim 6 into a host cell;
optionally, the recombinant immune cell further comprises a chimeric antigen receptor, the chimeric antigen receptor being the chimeric antigen receptor encoded by the second nucleic acid fragment as defined in the second nucleic acid molecule according to any one of claims 7 to 10;
optionally, the chimeric antigen receptor in the recombinant immune cell is expressed by introducing the second expression vector according to claim 11 into a host cell.

14. The recombinant immune cell according to claim 13, comprising at least one of an immune cell, a neuron, a progenitor cell or a precursor cell, an epithelial cell, an endothelial cell, or a stem cell;
optionally, the host cell comprises at least one of a T cell, a B cell, a monocyte, an NK cell, a dendritic cell, a macrophage, a regulatory T cell, a helper T cell, a cytotoxic T cell, an NKT cell, or a δγ T cell.

15. A pharmaceutical composition, comprising:
the recombinant immune cell according to any one of claims 12 to 14; and
optionally, a pharmaceutically acceptable excipient.

16. Use of the recombinant immune cell according to any one of claims 12 to 14 or the pharmaceutical composition according to claim 15 in the manufacture of a medicament for preventing and/or treating a disease or in the prevention and/or treatment of a disease;
optionally, the disease comprises cancer or a tumor, an autoimmune disease, inflammation, and a disease associated with cellular senescence.

17. The recombinant immune cell according to any one of claims 12 to 14 or the pharmaceutical composition according to claim 15, for use in the prevention and/or treatment of a disease;
optionally, the disease comprises cancer or a tumor, an autoimmune disease, inflammation, and a disease associated with cellular senescence.

18. A method for treating or preventing a disease, the method comprising:
administering to a subject a pharmaceutically acceptable amount of the recombinant immune cell according to any one of claims 12 to 14 or the pharmaceutical composition according to claim 15.
